(19)

Europäisches
Patentamt
European
Patent Office
Office européen
des brevets

(11) **EP 3 897 184 B1**

(12) **EUROPEAN PATENT SPECIFICATION**

(45) Date of publication and mention
of the grant of the patent:
**26.06.2024   Bulletin 2024/26**

(21) Application number: **19832842.9**

(22) Date of filing: **28.11.2019**

(51) International Patent Classification (IPC):
**A23J 1/20** *(2006.01)*          **G01N 33/68** *(2006.01)*
**A23L 33/19** *(2016.01)*

(52) Cooperative Patent Classification (CPC):
**A23J 1/205; A23L 33/19; G01N 33/6848;**
G01N 33/04; G01N 2333/4731

(86) International application number:
**PCT/EP2019/082863**

(87) International publication number:
**WO 2020/126385 (25.06.2020 Gazette 2020/26)**

(54) **DETERMINATION AND QUANTIFICATION OF PROTEOSE PEPTONE CONTENT AND/OR BETA-CASEIN CONTENT AND NUTRITIONAL COMPOSITON WITH REDUCED BETA-CASEIN DERIVED PROTEOSE PEPTONE CONTENT**

BESTIMMUNG UND QUANTIFIZIERUNG DES PROTEOSEPEPTONGEHALTS UND/ODER DES BETA-CASEINGEHALTS UND DER NAHRUNGSZUSAMMENSETZUNG MIT REDUZIERTEM BETA-CASEIN-ABGELEITETEN PROTEOSEPEPTONGEHALT

DÉTERMINATION ET QUANTIFICATION DE TENEUR EN PEPTONE DE PROTÉOSE ET/OU DE TENEUR EN BÊTA-CASÉINE ET COMPOSITION NUTRITIONNELLE AVEC TENEUR RÉDUITE EN BÊTA-CASÉINE ET PEPTONE DE PROTÉOSE DÉRIVÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **20.12.2018   EP 18214628**

(43) Date of publication of application:
**27.10.2021   Bulletin 2021/43**

(73) Proprietor: **Société des Produits Nestlé S.A.
1800 Vevey (CH)**

(72) Inventors:
 • **AFFOLTER, Michael
   1073 Savigny (CH)**
 • **ERDMANN, Peter
   3006 Bern (CH)**
 • **FUERER, Christophe
   1163 Etoy (CH)**
 • **O'REGAN, Jonathan
   Killarney, Co.Kerry V93E1T2 (IE)**

(74) Representative: **Gagliardi, Tatiana et al
Société des Produits Nestlé S.A.
Avenue Nestlé 55
1800 Vevey (CH)**

(56) References cited:
**EP-A1- 1 234 507          EP-A1- 2 098 122
EP-A1- 2 520 181          WO-A1-02/28413
US-A1- 2017 332 690**

- **DELPHINE VINCENT ET AL: "Quantitation and Identification of Intact Major Milk Proteins for High-Throughput LC-ESI-Q-TOF MS Analyses", PLOS ONE, vol. 11, no. 10, 17 October 2016 (2016-10-17), page e0163471, XP055596070, DOI: 10.1371/journal.pone.0163471 & Vincent: "Supplementary information for Vincent et al. 2016", PLoS One. 2016; 11(10): e0163471., 17 October 2016 (2016-10-17), pages 1-14, XP055596071, Retrieved from the Internet: URL:https://journals.plos.org/plosone/arti cle/file?type=supplementary&id=info:doi/10 .13 71/journal.pone.0163471.s001 [retrieved on 2019-06-12] & Delphine Vincent: "Supplementary information for Vincent et al. 2016", PLoS One. 2016; 11(10): e0163471., 17 October 2016 (2016-10-17), pages 10-25, XP055596437, Retrieved from the Internet: URL:https://journals.plos.org/plosone/arti cle/file?id=10.1371/journal.pone.0163471.s 002&type=supplementary [retrieved on 2019**
- **Nadia Sargaeva ET AL: "Application Note LC/Mass spectrometry from PerkinElmer: LC/MS Study of Casein Proteins in Milk", , 1 January 2014 (2014-01-01), pages 1-3, XP055596098, Retrieved from the Internet: URL:https://www.perkinelmer.com/lab-soluti ons/resources/docs/APP_CaseinProteinsinMil k.pdf [retrieved on 2019-06-13]**

- **CATTANEO STEFANO ET AL: "Targeted peptides for the quantitative evaluation of casein plasminolysis in drinking milk", FOOD CHEMISTRY, ELSEVIER LTD, NL, vol. 155, 27 January 2014 (2014-01-27), pages 179-185, XP028624342, ISSN: 0308-8146, DOI: 10.1016/J.FOODCHEM.2014.01.053**

Remarks:

The complete document including Reference Table(s) and the Sequence Listing(s) can be downloaded from the EPO website

**Description**

**Technical field of the invention**

The present invention relates to a method for determining and quantifying β-casein derived proteose peptones such as PP-5, PP8s and PP8f, and/or β-casein. The present disclosure also relates to a nutritional composition comprising a reduced content of β-casein derived proteose peptones such as PP-5, PP8s and PP8f.

**Background of the invention**

Milk from dairy cows has been regarded as nature's perfect food, providing an important source of nutrients including high quality proteins, carbohydrates and selected micronutrients. Milk proteins are primarily caseins with the remaining proteins being grouped into the group of whey proteins, the major of which are betalactoglobulin and alpha-lactalbumin. Among the caseins, β-casein is the second most abundant protein in cow's milk and has an excellent nutritional balance of amino acids. However, different mutations in the bovine β-casein gene have led to several genetic variants where the so-called A1 and A2 variants are the most common.

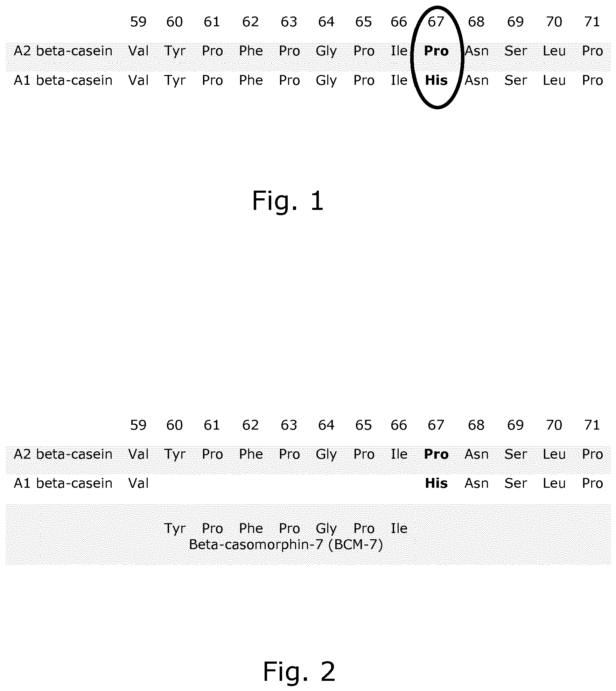

The A1 and A2 variants of β-casein differ at amino acid position 67 of the secreted β-casein protein, with histidine (His) in the A1 variant of β-casein and proline (Pro) in the A2 variant of β-casein, as a result of a single nucleotide difference. This is illustrated in Figure 1.

The A2 variant of β-casein is more comparable to human β-casein in terms of digestive breakdown. It has been suggested that the A1 variant of β-casein may be associated with cow's milk intolerance, and a statistically significant positive association between abdominal pain and stool consistency has been observed when participants consumed an A1 diet, but not an A2 diet (Ho et al, 2014).

The digestive difference observed between the A1 and A2 variants may be due to the particular polymorphism as described above. The polymorphism leads to a key conformational change in the secondary structure of the β-casein protein.

Gastrointestinal proteolytic digestion of the A1 (but not the A2) variant of β-casein leads to generation of β-casomorphin 7 (BCM-7) that is an exogenous opioid peptide (exorphin) that may activate opioid receptors throughout the body (EFSA Scientific Report, 2009). This is illustrated in Figure 2.

Originally, all cows produced milk containing only the A2 variant of β-casein, but genetic variation has resulted in mixed herds. Thus, cow herds that are typically used to produce milk normally produce a mixture of the A1 and A2 variants of β-casein but with different amount of A1 as compared to A2.

Proteolysis of β-casein by plasmin has been shown to lead to the generation of γ-caseins and proteose peptones (PP8 fast, PP8 slow and PP-5). The proteose peptones are not precipitated with caseins during acidification or enzymatic treatment with rennet and remain in the whey fraction (reviewed in Karamoko et al. 2013). PP8s slow and PP-5 correspond to sequences 29-105/7 and 1-105/7, respectively, and both include position 67. Thus, whey products derived from milk containing A1 β-casein will likely contain proteose peptones of the A1 type. The proteose peptones are derived from the same gene and occur through endogeonous events, why they are also considered as proteoforms of β-casein.

EP 2 098 122 discloses a composition enriched in proteose peptone fraction and depleted in β-lactoglobulin exhibiting a different protein composition than the proteose peptone fraction isolated from milk. EP1234507 A1 discloses a fraction derived from a whey protein fraction ("diary isolate") having "reduced" proteose peptones. The fraction is prepared from a whey protein fraction from which casein is removed by acidic precipitation, resulting in a reduced proteose peptones content.

Intact protein analyses by reverse-phase-high performance liquid chromatography (RP-HPLC) or capillary electrophoresis (CE) coupled to UV detection can effectively separate most β-casein variants in raw milk samples (see for instance de Jong et al., 1993, Visser et al., 1995, Bonfatti et al., 2008, Poulsen et al., 2016). In manufactured products however, processing conditions induce reactions between proteins and reducing sugars (Maillard reaction) that yield multiple proteoforms containing one or more sugar adducts (Fenaille et al., 2006). As a consequence, protein peaks split and broaden, creating overlaps that prevent the analysis of single proteoforms using UV detection (Vallejo-Cordoba, 1997, Feng et al., 2017).

The human Breast Milk (HBM) is known to contain the A2 form of human beta-casein. By definition, it represents a gold standard in terms of infant nutrition. There is therefore a need to provide (synthetic) nutritional compositions that mimic in the best possible manner the composition of HBM.

Due to the digestive differences observed in humans toward the A1 and A2 variants of cow β-casein, it would be advantageous to be able to detect and quantify in a simple and efficient way the amount of β-casein and its proteoforms. Currently, there is no simple and efficient method to characterize and quantify individual milk proteins and their proteoforms such as proteose peptones in finished products.

Furthermore, there is a need to provide a nutritional composition that comprises a relatively low amount or proportion of A1 β-casein or of β-casein derived proteose peptones or may be deprived of any form hereof.

Also, there is a need to provide a nutritional composition that comprises whey fractions, which are deprived or reduced in β-casein derived proteose peptones.

## Summary of the invention

An object of the present invention relates to an improved method for efficient and simple detection of β-casein derived proteose peptones as well as a method for reducing the content of β-casein derived proteose peptones.

It is a further object of the present disclosure (not part of the present invention) to provide a nutritional composition such as an infant formula, which may prevent abdominal pain and/or improve stool inconsistency of an infant.

It relates to a method for determining and quantifying β-casein derived proteose peptones, said method comprising the steps of

(i) providing a dairy-based product to be analysed;
(ii) subjecting said product using liquid chromatography - mass spectrometry analysis;
(iii) determining and/or quantifying said β-casein derived proteose peptones and/or β-casein in said product by detecting compounds of defined m/z values or deconvoluting one or more mass spectrometry spectra to calculate monoisotopic masses.

A first aspect of the invention relates to a method for producing a whey protein fraction having a reduced β-casein derived proteose peptone content, said method comprising the steps of

(i) providing a whey protein fraction;
(ii) determining and quantifying said β-casein derived proteose peptones in said whey protein fraction as described herein; and
(iii) selecting said whey protein fraction having at the most 10% by weight of β-casein derived proteose peptones based on the total protein in said whey protein fraction forming a selected whey protein fraction, or reducing the β-casein derived proteose peptone content in said whey protein fraction to a concentration of at the most 10% by weight based on total protein in the whey protein fraction forming a reduced whey protein fraction by gel filtration, wherein determining and quantifying said β-casein derived proteose peptones in said whey protein fraction comprising the steps of:

(i) providing a dairy-based product to be analysed;
(ii) subjecting said product using liquid chromatography - mass spectrometry analysis;
(iii) determining and/or quantifying said β-casein derived proteose peptones and/or β-casein in said product by detecting compounds of defined m/z values or deconvoluting one or more mass spectrometry spectra to calculate monoisotopic masses.

A second aspect of the invention relates to a method for producing a nutritional composition having a reduced β-casein derived proteose peptone content, said method comprising the steps of

(i) providing a selected whey protein fraction or a reduced whey protein fraction as claimed;
(ii) preparing said nutritional composition with a reduced β-casein derived proteose peptone content from said selected whey protein fractions, said reduced whey protein fractions or a mixture hereof.

It discloses (not forming part of the present invention) a whey protein fraction having a reduced β-casein derived proteose peptone content, obtainable by the method as described herein.

It discloses (not forming part of the present invention) a nutritional composition comprising said whey protein fraction having a reduced β-casein derived proteose peptone content as described herein or obtainable by the method as described herein.

It discloses (not forming part of the present invention) a nutritional composition as described herein for use as a medicament.

It discloses (not forming part of the present invention) an infant formula as described herein for use in treating, preventing and/or ameliorating abdominal pain in an infant and/or improving gut comfort in said infant.

It discloses (not forming part of the present invention) an infant formula as described herein for use in treating, preventing and/or ameliorating lactose intolerance in an infant.

It discloses (not forming part of the present invention) the use of the infant formula as described herein for improving the stool consistency.

**Brief description of the figures**

Figure 1 shows a partial sequence alignment between the sequence of A2 beta-casein and A1 beta-casein relating to the amino acid sequences 59-71. The amino acid at position 67 is highlighted demonstrating the one amino acid difference between A1 beta-casein and A2 beta-casein being His and Pro, respectively;

Figure 2 shows a partial sequence alignment between the sequence of A2 beta-casein and A1 beta-casein relating to the amino acid sequences 59-71. The one amino acid difference between the two beta-caseins influences the cleavage ability of the two strands as A2 beta-casein is not cleaved due to the presence of Pro at position 67, while A1 beta-casein is cleaved on the N-terminal side of His forming a possibility for a seven amino acid long sequence to be formed i.e. Beta-casomorphin-7 (BCM-7);

Figure 3 shows a description of the LC-HRMS method steps: (a) sample preparation, (b) deconvoluted spectra with monoisotopic masses for intact $\beta$-casein and (c) bubble chart representation of the intact $\beta$-casein region (bubble sizes represent signal intensities).

Figure 4 shows the global fingerprinting and the intact $\beta$-casein region. Major milk proteins were separated in a (a) global fingerprint of a raw milk sample. Different proteoforms were detected for (b) intact $\beta$-casein. A1, A2 and B are genetic variants, superscript notations correspond to the number of lactose (L) adducts;

Figure 5 shows examples of global fingerprinting in raw milk, human milk, infant formula (IF), skim milk powders (SMP) and whey samples;

Figure 6 shows bubble charts (intact $\beta$-casein region) of seven A2 infant formula samples either untreated or spiked at 5% with $\beta$-casein A1 (5 g of intact $\beta$-casein A1 / 100 g of total intact $\beta$-casein);

Figure 7 shows how glycation affects MS readings. (a) Bubble charts (intact $\beta$-casein region) showed increased lactosylation during solid-state glycation experiment. (b) The percentage of unglycated signal (black bar) decreased over time (c) shows the total $\beta$-casein signal decreases with increased glycation and (d) had a linear relationship with the percentage of unglycated signal. Plotting the signal reduction as a function of the percentage of unglycated signal (e) allowed calculating a glycation correction factor;

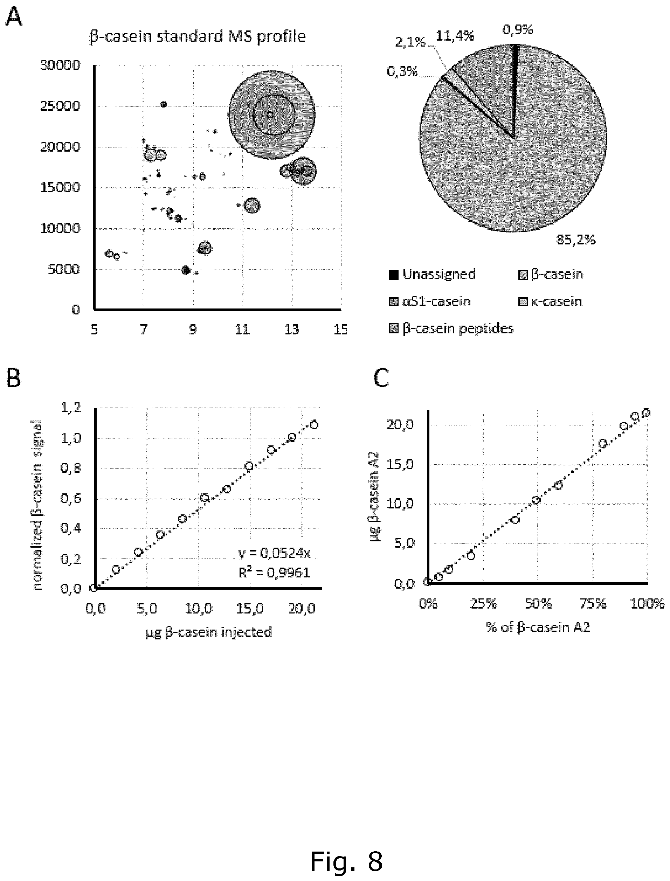

Figure 8 shows a characterization of the $\beta$-casein standard and establishment of the $\beta$-casein calibration curve. Analysis of the $\beta$-casein standard revealed that about 15% of total signal could not be attributed to intact $\beta$-casein (a). A linear calibration curve was established using normalized (to SMP, which was corrected for glycation) intact $\beta$-casein MS signal and adjusted $\beta$-casein amounts (b). Cross-mixing experiments between A1|A1 and A2|A2 skim milk samples indicated that the relationship remained linear even in a dairy matrix (c). The protein content was kept constant during the cross-mixing experiment;

Figure 9 shows the quantification of intact $\beta$-casein in infant formulas. $\beta$-casein was quantified in seven infant formula samples (a), where each sample was analyzed in triplicate five times. Theoretical values are based on recipe and generic milk protein composition. A further set of infant formulas (both A2-based or based on skim milk powder containing multiple genetic variants) was analyzed in triplicate in a single day (b). Hatched bars represent best guesses because the %whey of those samples was not indicated on the boxes;

Figure 10 shows the result of LC-HRMS analysis of intact proteins in different whey protein concentrates (WPC). The intact protein region is marked by the circle with the solid perimeter, the $\beta$-casein proteose peptones are included in the region marked by the circle with the dashed perimeter;

Figure 11 shows a close-up of the area shown in Figure 10 by the circle with the dashed perimeter demonstrating the content of proteose peptones. Black represents $\beta$-casein A2 derived proteose peptones, gray represents $\beta$-casein A1 derived proteose peptones and white represent unassigned compounds;

Figure 12 shows the result of LC-HRMS analysis of intact proteins in different finished products focusing on the area containing proteose peptones.

Figure 13 shows the correlation between measured PP5 signal for both PP5 A1 and PP5 A2 in relation to the percentage of A2 SMP in the sample. The protein content was kept constant during the cross-mixing experiment;

Figure 14 shows a schematic view of intact β-casein and products generated upon β-casein cleavage. The position of the tryptic peptides are shown by the horizontal arrows with the common peptides Tot1 and Tot2 as well as the specific peptides A1/2N, A1/2S and A1/2T. The Tot1 and Tot2 are at the C-terminal of β-casein where the sequence is identical for A1 and A2 and are not present in proteose peptones. The peptides A1/2N, A1/2S and A1/2T include the amino acid at position 67 that differs between A1 and A2 ;

Figure 15 shows the result obtained by LC-MS of a tryptic digest of different finished products including skimmed milk powder and several infant formulas with regard to the peptide A1S;

Figure 16 shows the result obtained by LC-MS of a tryptic digest of different finished products including skimmed milk powder and several infant formulas with regard to the peptide A1N;

Figure 17 shows the result obtained by LC-MS of a tryptic digest of different finished products including skimmed milk powder and several infant formulas with regard to the peptide A1T;

Figure 18 shows the result obtained by LC-MS of a tryptic digest of different finished products including skimmed milk powder and several infant formulas with regard to the peptide A2S;

Figure 19 shows the result obtained by LC-MS of a tryptic digest of different finished products including skimmed milk powder and several infant formulas with regard to the peptide A2N;

Figure 20 shows the result obtained by LC-MS of a tryptic digest of different finished products including skimmed milk powder and several infant formulas with regard to the peptide A2T;

Figure 21 shows the result of a tryptic digest of different finished products including skimmed milk powder and several infant formulas with regard to the peptide Tot1;

Figure 22 shows the result of a tryptic digest of different finished products including skimmed milk powder and several infant formulas with regard to the peptide Tot2; Figure 23 shows the result of a tryptic digest on skimmed milk powder, lactose, and different whey protein concentrates (WPC) measuring the amount of the Tot1 peptide using LC-MS;

Figure 24 shows the result of a tryptic digest on skimmed milk powder, lactose, and different whey protein concentrates (WPC) measuring the amount of the Tot2 peptide using LC-MS;

Figure 25 shows the result of a tryptic digest of skimmed milk powder, lactose and different whey protein concentrates (WPC) measuring the amount of the A1S peptide using LC-MS;

Figure 26 shows the result of a tryptic digest of skimmed milk powder, lactose and different whey protein concentrates (WPC) measuring the amount of the A1N peptide using LC-MS;

Figure 27 shows the result of a tryptic digest of skimmed milk powder, lactose and different whey protein concentrates (WPC) measuring the amount of the A1T peptide using LC-MS;

Figure 28 shows the result of a tryptic digest of skimmed milk powder, lactose and different whey protein concentrates (WPC) measuring the amount of the A2S peptide using LC-MS;

Figure 29 shows the result of a tryptic digest of skimmed milk powder, lactose and different whey protein concentrates (WPC) measuring the amount of the A2N peptide using LC-MS;

Figure 30 shows the result of a tryptic digest of skimmed milk powder, lactose and different whey protein concentrates (WPC) measuring the amount of the A2T peptide using LC-MS;

The present invention will now be described in more detail in the following.

## Detailed description of the invention

Definitions

Prior to discussing the present invention in further details, the following terms and conventions will first be defined: The term "LC" refers to "liquid chromatography" as known to the person skilled in the art. It should be emphasized that LC also includes HPLC i.e. high performance liquid chromatography and UPLC i.e. ultra performance liquid chromatography.

The term "MS" refers to "mass spectroscopy" as known to the person skilled in the art. It should be emphasized that MS also includes HRMS i.e. high resolution mass spectroscopy.

The term "compounds of defined m/z values" means that the MS is set to measure compounds having only m/z values specifically defined by the user when measuring on a given sample.

The term "A1/A1 cows" refers to cows with the homozygous genotype A1A1. Milk obtained from A1/A1 cows is denoted "A1 milk".

The term "A2/A2 cows" refers to cows with the homozygous genotype A2A2. Milk obtained from A2/A2 cows is denoted "A2 milk".

The term "A1/A2 cows" refers to cows with the heterozygous genotype A1A2. Milk obtained from A1/A2 cows is denoted "A1/A2 milk".

The term "A1 whey" refers to whey produced essentially from A1 milk.

The term "A2 whey" refers to whey produced essentially from A2 milk.

The term "A1/A2 whey" refers to whey either being produced from A1/A2 milk or being a mixture of A1 whey and A2 whey or being produced from a mixture of A1 milk and A2 milk, A1 milk and A1/A2 milk, A2 milk and A1/A2 milk or A1 milk, A2 milk and A1/A2 milk.

The term "A2 β-casein" refers to the A2 variant of bovine β-casein having the amino acid sequence according to SEQ ID NO. 1 (secreted form of protein). In the present context, other variants, which include a proline in position 67 may be included into A2 β-casein.

The term "A1 β-casein" refers to the A1 variant of bovine β-casein having the amino acid sequence according to SEQ ID NO. 2 (secreted form of protein). SEQ ID NO. 1 and SEQ ID NO. 2 only differ from each other in that A1 β-casein contains a histidine at position 67, whereas A2 β-casein contains a proline at position 67. In the present context, other variants, which include a histidine in position 67 may be included into A1 β-casein.

The term "intact β-casein" refers to the protein, which is not cleaved except for the removal of the signal sequence e.g. a protein as disclosed by SEQ ID NO. 1 and 2.

The term "β-casomorphin 7" also described as "BCM-7" refers to the peptide having the amino acid sequence Tyr-Pro-Phe-Pro-Gly-Pro-Ile.

The term "WPC" refers to "whey protein concentrate". In this context, WPC includes traditional WPC according to the USP definition as well as whey reduced in lactose and whey reduced minerals i.e. the protein level may be as low as 10% w/w.

The term "WPI" refers to "whey protein isolate" and is a whey protein concentrate having a whey protein content on a dry basis of not less than 90% by weight.

The term "whey protein fraction" refers to a composition comprising whey proteins e.g. WPC and/or WPI.

The term "standard SMP" refers to "standard skimmed milk powder" and is derived from milk obtained from mixed herds of cows and thus, comprises multiple variants of β-casein including A1 β-casein and A2 β-casein.

The term "A2 SMP" refers to "A2 skimmed milk powder" and comprises only A2 β-casein and not A1 β-casein.

The term "proteoform" refers to highly related protein molecules arising from all combinatorial sources of variation giving rise to products arising from a single gene. This includes products differing due to genetic variations, alternatively spliced RNA transcripts and post-translational modifications.

The term "proteose peptone" is the same as the one used in Swaisgood, 1982, i.e. the term "proteose peptone" refers to those proteins/peptides that remain in solution after milk has been heated at 95°C for 20 minutes and then acidified to pH 4.7 with 12% trichloro-acetic acid.

The term "β-casein derived proteose peptones" refers to proteose peptones derived from β-casein alone such as PP-5, PP-8 fast and PP-8 slow.

The term "proteose peptone 5", "PP5" or "PP-5" refers to the residues 1-105 and 1-107 derived from β-casein.

The term "proteose peptone 8 fast", "PP8f" or "PP8 fast" refers to the residues 1-28 derived from β-casein. PP8 fast may also be referred to as "bcas4P 1-28".

The term "proteose peptone 8 slow", "PP8s" or "PP8 slow" refers to the residues 29-105 and 29-107 derived from β-casein. PP8 slow may also be referred to as "bcas1P 29-105" and "bcas1P 29-107".

The term "infant" refers to a child under the age of 12 months; in one embodiment of the invention the term "infant" can be extended to include children at any age up to and including 18 months, or at any age up to and including 24 months.

The term "infant formula" as used herein refers to a foodstuff intended for particular nutritional use by infants during the first months of life (such as from 0 to 12 months, 0 to 10 months, 0 to 8 months, 0 to 6 months or 0 to 4 months) and satisfying by itself the nutritional requirements of this category of person (Article 2(c) of the European Commission Directive 91/321/EEC 2006/141/EC of 22 December 2006 on infant formulae and follow-on formulae). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula".

The term "follow-up formula" or "follow-on formula" refers to a formula that is given from the 6th month onwards. It constitutes the principal liquid element in the progressively diversified diet of this category of person.

The term "powder" in the present context means a dry, bulk solid composed of a large number of very fine particles that may flow freely when shaken or tilted. The powder may contain water in amounts not exceeding 10%, such as not exceeding 9%, 8%, 7%, 6%, 5%, 4%, 3%, 2%, 1.5%, 1% or 0.5%.

The term "nutritional composition" means a composition, which nourishes a subject. This nutritional composition is usually to be taken orally or intravenously. It may include a lipid or fat source, a carbohydrate source and/or a protein source. The nutritional composition can be in solid form (e.g. powder) or in liquid form.

In a particular embodiment, the composition is a hypoallergenic nutritional composition. The expression "hypoallergenic nutritional composition" means a nutritional composition that is unlikely to cause allergic reactions.

In a particular embodiment, the nutritional composition is a "synthetic nutritional composition". The expression "synthetic nutritional composition" means a mixture obtained by chemical and/or biological means, which can be chemically identical to the mixture naturally occurring in mammalian milks (i.e. the synthetic nutritional composition is not breast milk).

"Probiotic bacteria" means microbial cell preparations or components of microbial cells with a beneficial effect on the health or well-being of the host. A definition of probiotic bacteria is given in Salminen S et al. 1999;

"Prebiotic" means a selectively fermented ingredient that allows specific changes, both in the composition and/or activity in the gastrointestinal microflora that confers benefits upon host well-being and health. Prebiotica are discussed in Roberfroid MB 2007;

Detection and/or quantification of β-casein derived proteose peptones and/or β-casein

It discloses (not part of the present invention) a method for determining and/or quantifying β-casein derived proteose peptones and/or β-casein, said method comprising the steps of

(i) providing a dairy-based product to be analysed;
(ii) subjecting said product using liquid chromatography - mass spectrometry analysis;
(iii) determining and/or quantifying said β-casein derived proteose peptones and/or β-casein in said product by detecting compounds of defined m/z values or deconvoluting one or more mass spectrometry spectra to calculate monoisotopic masses.

The dairy-based product may be selected from a list of finished products such as infant formula, maternal nutrition, adult nutritionals or dairy products such as milk, milk powder, liquid whey, whey powder, caseinates, WPC, WPI.

The dairy-based product to be analysed is an infant formula or a whey protein fraction.

The dairy-based product to be analysed may be a powder or a liquid. In one embodiment, the product is a powder, which is dissolved prior to analysing the product. The powder is preferably dissolved by dispersing the powder in water. However, the powder may also be dissolved in other liquids such as a reducing agent as defined below, buffered solutions such as ammonium bicarbonate, tris, trisodium citrate, HEPES, TEAB (triethylammonium bicarbonate) or denaturing buffers as defined below and combinations hereof.

The powder is dissolved in a reducing agent and a buffered solution, and optionally a denaturing buffer is added subsequently. In a further embodiment, the powder is dissolved in a reducing agent and denaturing buffer, and optionally a buffered solution is added subsequently. In a still further embodiment, the powder is dissolved in a buffered solution and a denaturing buffer, and optionally a reducing agent is added subsequently.

The powders are dispersed to a concentration of at the most 10% w/v protein in the liquid, such as at the most 9.5% w/v protein in the liquid, like at the most 9% w/v protein in the liquid, such as at the most 8.5 w/v% protein in the liquid, like at the most 8% w/v protein in the liquid, such as at the most 7.5% w/v protein in the liquid, like at the most 7% w/v protein in the liquid, such as at the most 6.5% w/v protein in the liquid, like at the most 6% w/v protein in the liquid, such as at the most 5.5% w/v protein in the liquid, like at the most 5% w/v protein in the liquid, such as at the most 4.5% w/v protein in the liquid, like at the most 4% w/v protein in the liquid, such as at the most 3.5 w/v% protein in the liquid, like at the most 3% w/v protein in the liquid, such as at the most 2.5% w/v protein in the liquid, like at the most 2% w/v protein in the liquid, such as at the most 1.5% w/v protein in the liquid, like at the most 1% w/v protein in the liquid, such as at the most 0.5% w/v protein in the liquid.

After the dissolving of the samples or alternatively for dissolving the samples, they are denatured and reduced by means of a denaturing and reducing buffers such as urea, thiourea, guanidine HCl, DTT, beta-mercaptoethanol, TCEP.

The sample is dissolved in water and diluted further in a mixture of trisodium citrate, Guanidine-HCl and DTT.

The sample is also dissolved in Tris and Urea and diluted in ammonium bicarbonate and DTT.

Preferably, the samples are also cleared before being analysed. This may be performed by centrifugation. However, this may also be performed by filtration.

The product to be analysed is analysed using intact protein analysis where the proteins are intact when subjecting them to liquid chromatography and following mass spectrometry (LC-MS), such as liquid chromatography high resolution mass spectrometry (LC-HRMS). Using intact protein analysis makes is possible to distinguish between different proteoforms of β-casein and genetic variants of full-length β-casein including the A1 and A2 variants. Thus, measurement on intact protein from the products makes it possible to obtain a complete fingerprinting of the protein profile of the product. Also, the different genetic variants of proteose peptones may be distinguished using the intact protein analysis method.

Said product is analysed by peptide analysis comprising a step of enzymatic digest of said product prior to analysing it. Thus, before being subjected to an analysis of LC-MS the product is subjected to an enzymatic digest e.g by digesting the product by means of trypsin or GluC (Endoproteinase GluC [Straphylococcuc aureus Protease V8]).

Using enzymatic digestion makes it possible to determine if A1 β-casein is present in a product or if proteose peptones from A1 and/or A2 β-casein are. However, due to the set-up of the method involving digestion of the proteins as such it is not possible to distinguish between proteose peptones and full-length β-casein but only to distinguish between the A1 and A2 genetic variants. This may be done by detecting sequences covering the amino acid 67, which differs between the A1 and A2 variant. Thus, a complete fingerprinting protein profile would not be obtained but the presence of the A1 variant and possible precursors for the BCM-7 would be detected.

Said enzymatic digest is performed by tryptic digest or GluC digest.

The liquid chromatography is a high performance liquid chromatography. In a further embodiment, the liquid chromatography is an ultra performance liquid chromatography. In a still further embodiment, the liquid chromatography is a nano liquid chromatography.

Standard setting for running the liquid chromatography process as known to the person skilled in the art may be used in this measurement.

For the intact protein analysis, the samples may be separated on a C4 column using a gradient at 0.5 ml/min. Preferably a buffer containing trifluoroacetic acid is used. However, the gradient may vary from 0.1 ml/min to 1 ml/min, such as from 0.1 ml/min to 0.5 ml/min. Alternatively, the following buffers may be used for the LC separation such as water/methanol or water/acetonitrile possibly including formic acid, difluoroacetic acid and/or trifluoroacetic acid.

For the peptide analysis, the samples may be separated on a C18 column using a gradient at 75 μl/min. Preferably a buffer containing formic acid is used. However, the gradient may vary from 0.1 μl/min to 100 μl/min, such as from 0.4 μl/min to 85 μl/min. Alternatively, the following buffers may be used for the LC separation such as water/methanol or water/acetonitrile possibly including formic acid, difluoroacetic acid and/or trifluoroacetic acid.

The MS chromatograms may be recorded on machines such as Thermo Orbitrap Elite or Thermo Q-Exactive HF.

The MS chromatograms are recorded on a Thermo Orbitrap Elite (heater temperature: 60°C, sheath gas: 20, aux gas: 5, sweep gas: 0, spray voltage: 3.8 kV, capillary temperature: 320°C, S-lens RF level: 60%, mass range 400 to 2'000 m/z, resolution 240'000, AGC target: 1e6).

The MS chromatograms are recorded on a Thermo Q-Exactive HF (heater temp: 100 °C, sheath gas: 53, aux gas: 14, sweep gas: 3, spray voltage: 3.5 kV, capillary temp: 320 °C, S-lens RF level: 70%, mass range: 400 to 2'000 m/z, resolution: 240'000, AGC target: 1e6, maximum IT: 200 ms).

Said mass spectrometry analysis is a high resolution mass spectrometry analysis.

The HRMS acquisition is preferably performed at a resolution above 100'000, such as between 120'000 and 240'000.

According to one embodiment, the determining and quantifying said β-casein derived proteose peptones is performed by detecting compounds of defined m/z values as indicated. Hereby, the presence and amount of specific peptide sequences may be detected. This is possible as the enzymatic digestion would lead to specific peptides due to specific cleavage.

In one embodiment, the peptide method uses an untargeted (data dependent acquisition-DDA). In another embodiment, the peptide method uses an untargeted (data independent acquisition-DIA) approach.

In a further embodiment, the peptide method uses a PRM (parallel reaction monitoring) or a MRM (multiple reaction monitoring) method. The precursors may preferably be selected on the first quadrupole and fragmented and MS2 data recorded. In one embodiment, precursor selection may be done according to the peptide list described by SEQ ID NO. 3-10.

According to another embodiment, the mass spectrometry data obtained after the measurement using LC-MS for each sample are deconvoluted to calculate monoisotopic masses. Thus in one embodiment, the determining and quantifying said β-casein derived proteose peptones in said product is performed by deconvoluting one or more mass spectrometry spectra to calculate monoisotopic masses.

The deconvolution may be performed by commercially available software such as the Thermo BioPharma Finder.

In one embodiment, the deconvolution is performed by a sliding windows algorithm. In a further embodiment, the deconvolution is performed by a fixed windows algorithm.

In one preferred embodiment, the deconvolution was performed by using Thermo BioPharma Finder 1.0 software using the Xtract algorithm (S/N threshold: 3, relative abundance threshold: 1%, fit factor: 80%, remainder threshold: 25%, overlaps, charge states: 5 to 50, minimum intensity: 1, expected intensity error: 3, m/z: 600 to 2000, minimum number of detected charge states: 3) and sliding windows (time: 5 to 20 min, target average spectrum width: 0.1 min, target average spectrum offset: 50%, merge tolerance: 1.5 Da, maximum RT gap: 0.5 min, minimum number of detected intervals: 3, XIC).

Deconvoluted monoisotopic masses were compared to a protein database containing the major milk protein components αS1-CN, oS2-CN, β-CN, κ-CN, γ-CN, α-lactalbumin, β-lactoglobulin, CGMP and β-casein proteose peptones. Combinatorial addition of standard protein modifications (phosphorylation, oxidation, lactosylation, glycosylation, and pyroglutamic acid) were tested to identify the majority of signals.

One way of quantifying the β-casein derived proteose peptones is to compare the signal intensities obtained by the measurements to a standard curve derived from measurements of known amounts of the different proteose peptones.

Hereby, the absolute amount of the proteose peptones in a given sample may be calculated and the amount quantified.

Alternatively, the signal intensity of the proteose peptone of interest may be expressed in terms of relative terms such as a percentage of the total amount of either protein in the sample or as a percentage of the β-casein content.

The compounds to be determined and quantified are β-casein derived proteose peptones.

In one embodiment, the proteose peptones are A1 β-casein derived proteose peptones. In a further embodiment, the β-casein derived proteose peptones are PP8 fast, PP8 slow and/or PP-5. In a still further embodiment, the A1 β-casein derived proteose peptones are PP8 fast, PP8 slow and/or PP-5.

Whey protein fraction having reduced β-casein derived proteose peptone content (selected whey protein fraction)

In a first aspect, this invention relates to a method for producing a whey protein fraction having a reduced β-casein derived proteose peptone content, said method comprising the steps of

(i) providing a whey protein fraction;
(ii) determining and quantifying said β-casein derived proteose peptones in said whey protein fraction as described herein; and
(iii) selecting said whey protein fraction having at the most 10% by weight of β-casein derived proteose peptones based on the total protein in said whey protein fraction forming a selected whey protein fraction, or reducing the b-casein derived proteose peptone content in said whey protein fraction to a concentration of at the most 10% by weight based on total protein in the whey protein fraction forming a reduced whey protein fraction by gel filtration,

Wherein determining and quantifying said β-casein derived proteose peptones in said whey protein fraction comprising the steps of:

(i) providing a dairy-based product to be analysed;
(ii) subjecting said product using liquid chromatography - mass spectrometry analysis;
(iii) determining and/or quantifying said β-casein derived proteose peptones and/or β-casein in said product by detecting compounds of defined m/z values or deconvoluting one or more mass spectrometry spectra to calculate monoisotopic masses.

Available whey protein fractions to be used in finished products such as nutritional compositions including infant formulas often comprises A1 whey and/or A1A2 whey due to standard production methods. However, this has not previously be considered an issue e.g. for the manufacturing of A2 infant formula, as A1 β-casein would be precipitated during the preparation of the whey and thus, not be part of the whey protein fraction. However, as shown in the examples, proteose peptones derived from A1 β-casein is detected in whey protein fractions such as WPC.

In one embodiment, the whey protein fraction is based upon A1 whey and/or A1A2 whey

Due to the benefits of the method as described above for determination and quantification of β-casein variants and proteoforms the content of β-casein derived protease peptones may be easily detected and quantified. Hereby, the whey protein fractions may be separated into whey protein fractions containing different amounts of protease peptones. Thus, whey protein fractions may be selected, which comprises at the most 10% by weight of protease peptones out of the total amount of proteins in the whey protein fraction.

In one embodiment, the selected whey protein fraction has a β-casein derived protease peptone content, such as a β-casein derived protease peptone content, of at the most 9.5% by weight, such as at the most 9% by weight, preferably at the most 8.5% by weight, such as at the most 8% by weight, more preferably at the most 7.5% by weight, such as at the most 7% by weight, even more preferably at the most 6.5% by weight, such as at the most 6% by weight, still more preferably at the most 5.5% by weight, such as at the most 5% by weight, most preferably at the most 4.5% by weight, such as at the most 4% by weight, at the most 3.5% by weight, such as at the most 3% by weight, preferably at the most 2.5% by weight, such as at the most 2% by weight, more preferably at the most 1.5% by weight, such as at the most 1% by weight, even more preferably at the most 0.75% by weight, such as at the most 0.50% by weight, still more preferably at the most 0.25% by weight, such as at the most 0.10% by weight, most preferably at the most 0.05% by weight, such as at the most 0.01% by weight, based on total protein in the whey protein fraction.

The total amount of protein in the whey protein fraction is preferably measured by Kjeldahl analyses (ISO 8968-1:2014).

In one embodiment, the protease peptones are PP8 fast, PP8 slow and/or PP-5. In a further embodiment, the protease peptones are PP8 slow and/or PP-5.

In one embodiment, the selected whey protein fraction has a PP8 slow and/or PP-5 content, of at the most 9.5% by weight, such as at the most 9% by weight, preferably at the most 8.5% by weight, such as at the most 8% by weight, more preferably at the most 7.5% by weight, such as at the most 7% by weight, even more preferably at the most 6.5% by weight, such as at the most 6% by weight, still more preferably at the most 5.5% by weight, such as at the most 5% by weight, most preferably at the most 4.5% by weight, such as at the most 4% by weight, at the most 3.5% by weight, such as at the most 3% by weight, preferably at the most 2.5% by weight, such as at the most 2% by weight, more preferably at the most 1.5% by weight, such as at the most 1% by weight, even more preferably at the most 0.75% by weight, such as at the most 0.50% by weight, still more preferably at the most 0.25% by weight, such as at the most 0.10% by weight, most preferably at the most 0.05% by weight, such as at the most 0.01% by weight, based on total protein in the whey protein fraction.

In a still further embodiment, the protease peptone is PP-5.

The present disclosure also relates to (but is not part of the present invention) a method for producing a whey protein fraction having a reduced β-casein derived protease peptone content, said method comprising the steps of

(i) providing a whey protein fraction has an initial content of protease peptones;
(ii) reducing the β-casein derived protease peptone content in said whey protein fraction to a concentration of at the least 5x less than the initial content of said whey protein fraction obtaining a reduced whey protein fraction, such as at least 8x less, like at the least 10x less, such as at the least 15x less than the initial content of said whey protein fraction.

In one embodiment, said whey protein fraction is based upon A1 whey and/or A1A2 whey.

By means of example, the at least 5x less is to be understood as if for example the initial content is 5 μg/mg then a 5x (five times) reduction would mean that the reduced whey protein fraction contains at the most 1 μg/mg and similarly if the signal intensity is 500 000 000 for the initial content then the signal intensity would be at the most 100 000 000 for the reduced whey protein fraction.

The reduction of at least 5x less, 8x less, 10x less or 15x less may be measured by means of the signal intensity obtained by the method for determining and quantifying as described herein.

In one embodiment, the whey protein fraction is tested using the method as described herein for determining and quantifying the content of protease peptones in order to decide whether or not the content of protease peptones needs to be reduced or not before the whey protein fractions is to be used.

The content of the β-casein derived protease peptones, in the whey protein fraction is reduced to at the most 10% by weight based on the total protein in the whey protein fraction.

The total amount of protein in the whey protein fraction is preferably measured by Kjeldahl analyses (ISO 8968-1:2014).

However, the protease peptone content may also be reduced by means of ion exchange chromatography, affinity chromatography or separation over membranes.

In a further embodiment, the final content of protease peptones in the reduced whey protein fraction may be determined and quantified by the method as described above.

In one embodiment, the reduced β-casein derived whey protein fraction has a proteose peptone content of at the most 9.5% by weight, such as at the most 9% by weight, preferably at the most 8.5% by weight, such as at the most 8% by weight, more preferably at the most 7.5% by weight, such as at the most 7% by weight, even more preferably at the most 6.5% by weight, such as at the most 6% by weight, still more preferably at the most 5.5% by weight, such as at the most 5% by weight, most preferably at the most 4.5% by weight, such as at the most 4% by weight, at the most 3.5% by weight, such as at the most 3% by weight, preferably at the most 2.5% by weight, such as at the most 2% by weight, more preferably at the most 1.5% by weight, such as at the most 1% by weight, even more preferably at the most 0.75% by weight, such as at the most 0.50% by weight, still more preferably at the most 0.25% by weight, such as at the most 0.10% by weight, most preferably at the most 0.05% by weight, such as at the most 0.01% by weight, based on total protein in the whey protein fraction.

In one embodiment, the proteose peptones are PP8 fast, PP8 slow and/or PP-5.

In a further embodiment, the proteose peptones are PP8 slow and/or PP-5.

In one embodiment, the reduced whey protein fraction has a PP8 slow and/or PP-5 content of at the most 9.5% by weight, such as at the most 9% by weight, preferably at the most 8.5% by weight, such as at the most 8% by weight, more preferably at the most 7.5% by weight, such as at the most 7% by weight, even more preferably at the most 6.5% by weight, such as at the most 6% by weight, still more preferably at the most 5.5% by weight, such as at the most 5% by weight, most preferably at the most 4.5% by weight, such as at the most 4% by weight, at the most 3.5% by weight, such as at the most 3% by weight, preferably at the most 2.5% by weight, such as at the most 2% by weight, more preferably at the most 1.5% by weight, such as at the most 1% by weight, even more preferably at the most 0.75% by weight, such as at the most 0.50% by weight, still more preferably at the most 0.25% by weight, such as at the most 0.10% by weight, most preferably at the most 0.05% by weight, such as at the most 0.01% by weight, based on total protein in the whey protein fraction.

In a still further embodiment, the proteose peptone is PP-5.

Nutritional composition such as an infant formula having reduced proteose peptone content

In another aspect, the invention relates to a method for producing a nutritional composition having a reduced β-casein derived proteose peptone content, said method comprising the steps of

(i) providing a selected whey protein fraction or a reduced whey protein fraction as described in claim 1;
(ii) preparing said nutritional composition with a reduced proteose peptone content from said selected whey protein fractions, said reduced whey protein fractions or a mixture hereof.

It discloses (but is not part of the present invention) a whey protein fraction having a reduced β-casein derived proteose peptone content, obtainable by the method as described herein.

It discloses ( but is not part of the present invention) a nutritional composition comprising said whey protein fraction having a reduced β-casein derived proteose peptone content as described herein or is obtainable by the method as described herein.

he β-casein derived proteose peptone content of the nutritional composition such as an infant formula is at the most 9% by weight based on total protein in the nutritional composition.

The total amount of protein in the whey protein fraction is preferably measured by Kjeldahl analyses (ISO 8968-1:2014).

The β-casein derived proteose peptone content of the nutritional composition is at the most 8.5% by weight, more preferably at the most 8% by weight, such as at the most 7.5% by weight, even more preferably at the most 7% by weight, such as at the most 6.5% by weight, still more preferably at the most 6% by weight, such as at the most 5.5% by weight, most preferably at the most 5% by weight, such as at the most 4.5% by weight, like at the most 4% by weight, at the most 3.5% by weight, more preferably at the most 3% by weight, such as at the most 2.5% by weight, even more preferably at the most 2% by weight, such as at the most 1.5% by weight, still more preferably at the most 1% by weight, such as at the most 0.75% by weight, most preferably at the most 0.50% by weight, such as at the most 0.25% by weight, like at the most 0.1% by weight, such as at the most 0.05% by weight, like at the most 0.01% by weight, based on total protein in the whey protein fraction.

The nutritional composition may be intended for any mammal, such as for example humans, and pets such as cats and dogs. In a preferred embodiment, the mammal is a human.

Examples of nutritional compositions are infant formula, maternal nutrition, adult nutritionals or dairy products.

In one embodiment, the nutritional composition is an infant formula.

The general composition of a nutritional composition such as an infant formula for use according to the present disclosure may optionally contain substances which may have a beneficial effect such as probiotic bacteria, fibres, lactoferrin, nucleotides, nucleosides, and/or the like in the amounts such as those customarily found in nutritional compositions to be fed to infants.

The probiotic bacteria may be selected from the group consisting of Lactobacillus such as Lactobacillus rhamnosus, Lactobacillus paracasei and Lactobacillus reuteri and Bifidobacterium such as Bifidobacterium lactis, Bifidobacterium breve and Bifidobacterium longum.

The nutritional composition, such as the infant formula, may optionally further comprise prebiotics, such as nondigestable carbohydrates that promote the growth of probiotic bacteria in the gut.

In a preferred disclosure, nutritional composition, such as the infant formula, comprises prebiotics selected from the group consisting of fructooligosaccharides (FOS), raftilose, inulin, raftiline, lactulose, cows' milk oligosaccharides (CMOS) and galactooligosaccharides (GOS).

The nutritional composition may also contain all vitamins and minerals understood to be essential in the daily diet in nutritionally significant amounts.

Thus a preferred disclosure relates to a nutritional composition, such as an infant formula, further comprising vitamins.

Minimum requirements have been established for certain vitamins and minerals. Examples of minerals, vitamins and other nutrients optionally present in the nutritional composition include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form.

If necessary, the nutritional composition, such as the infant formula, may contain emulsifiers and stabilizers such as soy lecithin, citric acid esters of mono- and diglycerides, and the like. This is especially the case if the composition is provided in liquid form.

A preferred disclosure relates to a nutritional composition, such as an infant formula, wherein at least one source of carbohydrates is selected form the group consisting of lactose, corn syrup solids, fructose, glucose, maltodextrins, dried glucose syrups, sucrose, trehalose, galactose, maltose, honey powders, starch, oligosaccharides, raftiline and raftilose.

Another preferred disclosure relates to a nutritional composition, such as an infant formula, further comprising anhydrous milk fat.

A yet other preferred disclosure relates to a nutritional composition, such as an infant formula, further comprising LC-PUFAs such as DHA, EPA, DPA and/or ARA.

The nutritional composition, such as the infant formula, may further comprise flavours such as but not limited to vanillin.

A particular preferred disclosure relates to a nutritional composition, such as an infant formula, further comprising fructo-oligosaccharides such as raftiline and/or raftilose.

The nutritional composition, such as the infant formula, may be a liquid formula or a powder that is to be reconstituted before use. A preferred disclosure is an infant formula being a powder.

In a preferred disclosure the nutritional composition is a liquid composition that has been prepared by reconstituting a powder.

The present disclosure further provides a nutritional composition (not part of the present invention), such as an infant formula, for use as complementary feeding of infants in association with human breast milk.

According to particular disclosure the infant is at an age between 0-12 months, preferably at an age between 0-6 months.

Therapeutic and non-therapeutic uses of a nutritional composition or infant formula having a reduced proteose peptone content.

The nutritional composition as described herein can be used as a medicament.

The infant formula as described herein relates to the use in treating, preventing and/or ameliorating abdominal pain in an infant.

The infant formula as described herein relates to the use in treating, preventing and/or ameliorating lactose intolerance in an infant.

It relates to the use of the infant formula as described herein for improving the stool consistency.

BCM-7 is known to affect the opioid receptors and is related to the stool consistency and abdominal pain. Thus, an infant formula comprising none or only a very limited amount of BCM-7 precursors i.e. proteose peptones PP-5 and PP8 slow derived from A1 β-casein, would result in less BCM-7 formed during the consumption of the infant formula and thus, less disadvantageous influence on the stool consistency and abdominal pain.

Furthermore, it is believed that administration of an infant formula of containing less precursor molecules of the BCM-7 and consequently resulting in less BCM-7 formed will result in less influence on opioid receptors being affected preventing lactose intolerance later in that infant's life. There is growing evidence that events early in life can precipitate disease, even though the disease may only manifest later in life.

In this context, treating is to be understood as a situation where a disorder is established, in contrast to preventing which takes place prior to a disorder is established. Ameliorating is to be understood as a situation where a disorder is established but not treated in a manner which results in the disorder disappearing but mere improves the health conditions of the individual suffering from the disease.

It discloses (not part of the present invention) a method of treating, preventing and/or ameliorating abdominal pain in an infant.

It discloses (not part of the present invention) a method of treating, preventing and/or ameliorating lactose intolerance in an infant.

The invention will now be described in further details in the following non-limiting examples.

## Examples

### Material and methods

#### Chemical reagents and samples

Guanidine hydrochloride (RDD001), trisodium citrate dihydrate (S1804), DL-dithiothreitol (43819) and β-casein standard (C6905) were purchased from Sigma-Aldrich (St. Louis, MI, USA), TFA (1.08178.0050) from VWR International (Radnor, PA, USA), and LC-MS grade water (1.15333.1000) and acetonitrile (1.00029.1000) from Merck (Darmstadt, Germany).

#### Milk samples

Raw milk was collected from the Teagasc Moorepark dairy farm (Fermoy, Co. Cork, Ireland) from cows that were genetically profiled as A1/A1, defatted, pasteurized using a Microthermics (UHT/HTST Electric Model 25HV Hybrid, Liquid Technologies, Wexford, Ireland) unit heated to 85°C and held for 23 s, followed by homogenization [GEA Niro Soavi S.p.A. Type: NS2006H (non-aseptic)] using 2-stage homogenization at a total homogenization pressure of 2500 psi. The sample was spray dried to produce a skimmed milk powder sample referred to as A1|A1 SMP. Commercially available A1/A2 and A2/A2 SMP were purchased from Europe and the USA, buffalo SMP was purchased from India, and pasteurized breast milk from pooled human donors (991-01-P) was purchased from Lee Biosolutions (Maryland Heights, MO USA). Different batches, either from the same or different manufacturers, are numbered A, B, C etc.

#### Finished products and whey protein concentrates

Commercially available infant formula powders suitable for infants (0-12 months) and children (1 year and above) were obtained from different manufacturers and for different age groups. The different samples were denoted either IF1-IF22 (see Table 1) or IFa-IFj in the experiments. In this regard, it should be noted that IFa=IF1, IFb=IF2, IFc=IF3, IFd=IF4, IFe=IF5, IFf=IF6 and IFg=IF7.

**Table 1**

| ID | Type | Stage | %protein | %casein* | %β-casein** |
|---|---|---|---|---|---|
| IF1 | A2 | 1 | 10.2 | 35 | 1.19 |
| IF2 | A2 | 2 | 15.3 | 60 | 3.06 |
| IF3 | A2 | 3 | 13.9 | 60 | 2.78 |
| IF4 | A2 | 4 | 17.0 | 60 | 3.40 |
| IF5 | A2 | 1 | 10.4 | 30 | 1.04 |
| IF6 | A2 | 2 | 15.0 | 50 | 2.50 |
| IF7 | A2 | 3 | 15.0 | 60 | 3.00 |
| IF8 | A1/A2 | 1 | 10.6 | 40 | 1.41 |
| IF9 | A1/A2 | 2 | 14.9 | 40§ | 1.99 |
| IF10 | A1/A2 | 3 | 15.3 | 70§ | 3.57 |
| IF11 | A1/A2 | 3 | 22.2 | 78 | 5.77 |
| IF12 | A1/A2 | 1 | 11.0 | 40 | 1.47 |

(continued)

| ID | Type | Stage | %protein | %casein* | %β-casein** |
|---|---|---|---|---|---|
| IF13 | A1/A2 | 1 | 10.2 | 35 | 1.25 |
| IF14 | A1/A2 | 2 | 15.3 | 60 | 3.02 |
| IF15 | A1/A2 | 3 | 15.0 | 60 | 3.07 |
| IF16 | A1/A2 | 4 | 17.0 | 60 | 3.48 |
| IF17 | A2 | 1 | 9.9 | 30 | 1.01 |
| IF18 | A2 | 2 | 10.4 | 30 | 1.03 |
| IF19 | A2 | 3 | 12.8 | 50 | 1.94 |
| IF20 | A2 | 1 | 10.1 | 35 | 1.32 |
| IF21 | A2 | 2 | 11.7 | 60 | 2.41 |
| IF22 | A2 | 3 | 15.2 | 60 | 3.12 |
| * as indicated on the label (except in §, which are based on assumptions), ** calculated by multiplying the protein content by the casein content and dividing by 3 (as stated in Swaisgood, 1995) | | | | | |

Three different commercially available batches (denoted A, B and C) of WPC were tested. WPC35 is a whey protein concentrate with 35% w/w protein.

Two different commercially available batches of WPC28 (denoted A and B) were tested. WPC28 is demineralized WPC with 28% w/w protein.

WPC80 is a commercially available whey protein concentrate with 80% w/w protein which is alpha-lactalbumin enriched.

Sample preparation

Powders were dispersed to 3.5 % (w/v) protein in water using a volumetric flask, stirred for at least 30 min at room temperature, denatured with 4 volumes of denaturing buffer (Guanidine HCl 7.5 M, trisodium citrate 6.25 mM, DTT 23 mM), incubated at room temperature for 30 min, and cleared by centrifugation at 16'000 g for 10 min.

Trypsin digestion of intact proteins

The sample powders were dispersed to 3.5 % (w/v) protein in Tris 50 mM + 6M urea by mixing on an orbital shaker for one hour.

200 μL sample where diluted with 1000 μL ammonium bicarbonate 100 mM and vortex'ed. 10 μL of DTT (45 mM) was added to 100 μL of the diluted solution and incubated at 60 °C for 30 minutes. The sample was then cooled to room temperature before quickly spinning the sample and adding 10 μL iodoacetamide (100 mM). The sample was then incubated for 30 minutes at room temperature in the dark before adding 6 μL trypsin (0.2 μg/μL) and incubating overnight at 37 °C. The tryptic digestion is stopped by adding 6 μL formic acid 10%. The digested sample is centrifuged at 14'000g for 10 minutes before transferring the liquid phase into a vial for injection and determination via LC-MS.

LC-MS analysis

Cleared samples were analyzed by LC-MS based on existing methods (Bonfatti et al., 2008, Frederiksen et al., 2011).

For the intact protein analysis, samples were separated on a C4 column (Acquity UPLC Protein BEH C4, 300 Å, 1.7 μm, 2.1 mm x 150 mm) using the following gradient at 0.5 ml/min (Table 2):

**Table 2**

| Time (min) | 0 | 4 | 7 | 22 | 23 | 24 | 25 | 30 |
|---|---|---|---|---|---|---|---|---|
| %Buffer B | 15 | 35 | 42.5 | 52.5 | 80 | 80 | 15 | 15 |
| Buffer A: 0.1 % trifluoroacetic acid (TFA) in water, Buffer B: 0.1 % TFA in acetonitrile:water 90:10 | | | | | | | | |

The MS signal was recorded from 4 to 20 min in Full MS mode on a Thermo Q-Exactive HF (heater temp: 100 °C, sheath gas: 53, aux gas: 14, sweep gas: 3, spray voltage: 3.5 kV, capillary temp: 320 °C, S-lens RF level: 70%, mass range: 400 to 2'000 m/z, resolution: 240'000, AGC target: 1e6, maximum IT: 200 ms).

For the peptide analysis i.e. tryptic digest or GluC digest, the peptides were separated on a C18 column (Acquity UPLC BEH C18, 130 Å, 1.7 μm, 1.0 x 150 mm) using the following gradient at 75 μL/min (Table 3):

**Table 3**

| Time (min) | 0 | 30 | 31 | 33 | 35 | 45 |
|---|---|---|---|---|---|---|
| %Buffer B | 2 | 60 | 100 | 100 | 2 | 2 |

The MS signal was recorded from 3.5 to 35 min in PRM mode on a Thermo Q-Exactive HF (heater temp: 30 °C, sheath gas: 8, aux gas: 0, sweep gas: 0, spray voltage: 3.6 kV, capillary temp: 320 °C, S-lens RF level: 55, default charge: 2, MS2 resolution: 30'000, AGC target: 1e5, maximum IT: 100 ms, isolation window: 1.5 m/Z, isolation offset: 0.5 m/z).

The inclusion list was as shown in Table 4:

**Table 4**

| Name | Mass [m/z] | Formula [M] | SEQ ID NO |
|---|---|---|---|
| A1N | 884.95449 | AQTQSLVYPFPGPIHN | 3 |
| A2N | 864.95141 | AQTQSLVYPFPGPIPN | 4 |
| Tot2 | 415.72960 | AVPYPQR | 5 |
| A1S | 755.06013 | IHPFAQTQSLVYPFPGPIHN | 6 |
| A1T | 1072.17772 | IHPFAQTQSLVYPFPGPIHNSLPQNIPPLTQTPVVVPPFLQPEVMGVSK | 7 |
| A2S | 1112.08349 | IHPFAQTQSLVYPFPGPIPN | 8 |
| A2T | 1329.96880 | IHPFAQTQSLVYPFPGPIPNSLPQNIPPLTQTPVWPPFLQPEVMGVSK | 9 |
| Tot1 | 390.75254 | VLPVPQK | 10 |

## Data deconvolution

Raw data files were deconvoluted using Thermo BioPharma Finder 1.0 software using the Xtract algorithm (S/N threshold: 3, relative abundance threshold: 1%, fit factor: 80%, remainder threshold: 25%, overlaps, charge states: 5 to 50, minimum intensity: 1, expected intensity error: 3, m/z: 600 to 2000, minimum number of detected charge states: 3) and sliding windows (time: 5 to 20 min, target average spectrum width: 0.1 min, target average spectrum offset: 50%, merge tolerance: 1.5 Da, maximum RT gap: 0.5 min, minimum number of detected intervals: 3, XIC). Monoisotopic masses, total signal intensities and apex RTs were exported as csv files and used for the next steps.

Limit of detection experiments were also performed by deconvoluting raw data files using a fixed window centered around the β-casein peak. Parameters were as follows: S/N threshold: 1, relative abundance threshold: 1%, fit factor: 25%, remainder threshold: 25%, overlaps, charge states: 12 to 30, minimum intensity: 1, expected intensity error: 3, m/z: 800 to 2000, minimum number of detected charge states: 3, time: 11 to 13.5 min, relative intensity threshold: 1%. Monoisotopic masses and total signal intensities were used for the next steps.

## Proteoform attribution

Deconvoluted monoisotopic masses were compared to a protein database containing the major milk protein components αS1-CN, oS2-CN, β-CN, κ-CN, γ-CN, α-lactalbumin, β-lactoglobulin, CGMP and proteose peptones using a Nestlé developed software (Protein Analyzer). Combinatorial addition of standard protein modifications (phosphorylation, oxidation, lactosylation, glycosylation, and pyroglutamic acid) were tested to identify the majority of signals. Misattributed signals were corrected by manual verification. Total signal intensities were extracted for the relevant proteins or proteoforms (i.e. total β-casein, β-casein lactosylation states, β-casein genetic variants etc.). For clarity, only β-casein genetic variants A1, A2 and B are detailed in the Figures. β-casein I/H2 co-elutes with β-casein and was merged with β-casein A2. Both variants have a proline at position 67 and belong to the A2 type.

Solid-state protein glycation

The solid-state glycation experiment was based on Fenaille, 2003 (Fenaille et al., 2003). Briefly, 45 g of milk powder were incubated in an enclosure saturated with dipotassium carbonate for 8-10 days to reach 5.4 % humidity (initial % was 3.8 %).

Nine 2.1 g-aliquots of the humidified powder were incubated in 25-mL glass tubes at 60 °C in an oven for 45 min to 24 h. Glycation was stopped by transferring the tubes on ice for 5 min. Then, 20 g of water were added to obtain a 3.5 % (w/v) protein solution, which was mixed at room temperature for 1 h 30 on a roller mixer. Solutions were stored at 4°C overnight until the end of the experiment and warmed up for 2 h at RT before analysis. Samples were prepared as described above except that the incubation step was 10 min at 60°C on a thermomixer (650 rpm). MS chromatograms were recorded on a Thermo Orbitrap Elite (heater temperature: 60 °C, sheath gas: 20, aux gas: 5, sweep gas: 0, spray voltage: 3.8 kV, capillary temperature: 320 °C, S-lens RF level: 60 %, mass range: 400 to 2'000 m/z, resolution: 240'000, AGC target: 1e6).

β-casein quantification

A skim milk powder (SMP) sample was injected multiple times throughout each analytical series to normalize the signals across experiments.

An external calibration curve was established using β-casein standard adjusted for protein purity (see text). β-casein signal intensities were normalized with the average β-casein signal of the SMP samples (corrected for glycation, see above). The calibration curve was forced to 0 (y = 0.0524·x, where x is expressed in μg β-casein injected and y is dimensionless).

For each sample, β-casein intensity values were first corrected for glycation and normalized with the average β-casein signal of the SMP samples (corrected for glycation). Then the amount of β-casein injected was calculated through the external calibration curve. This calculation can be done for total β-casein, for a given variant e.g. A1 β-casein, or for a set of variants (β-casein A2 results also include the related I/H2 variant).

Method performance was evaluated by two operators in seven infant formula samples (stages 1-4, manufactured using skim milk powder of the A2 type) using triplicate injections on five different days.

**Example 1** - **LC-MS method, data output, deconvolution and visualization**

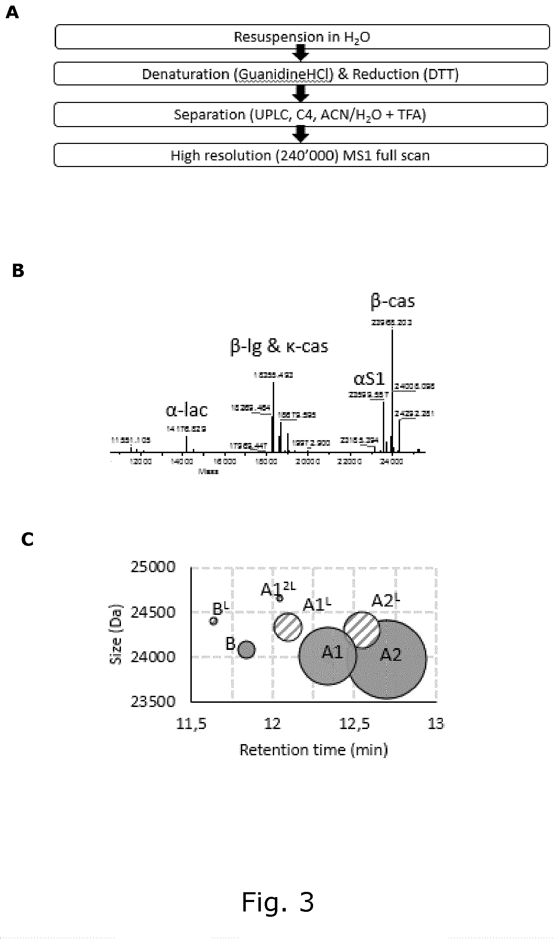

To analyze intact proteins, infant formulas and skim milk powder samples were dispersed in water to 3.5% (v/w) protein, denatured in 6 M guanidine, reduced with DTT, and cleared by centrifugation. Intact proteins were separated by UPLC on a C4 column using a water/ACN gradient with 0.1 % TFA and analyzed by high resolution mass spectrometry (Figure 3A). Chromatograms are sequentially deconvoluted using sliding windows and the Xtract algorithm to obtain the monoisotopic masses of the proteins (Figure 3B). Finally, proteoforms are visualized on bubble charts, with their retention time on the X-axis, monoisotopic size on the Y-axis, and signal intensity as bubble area (Figure 3C).

**Example 2** - **Global fingerprinting**

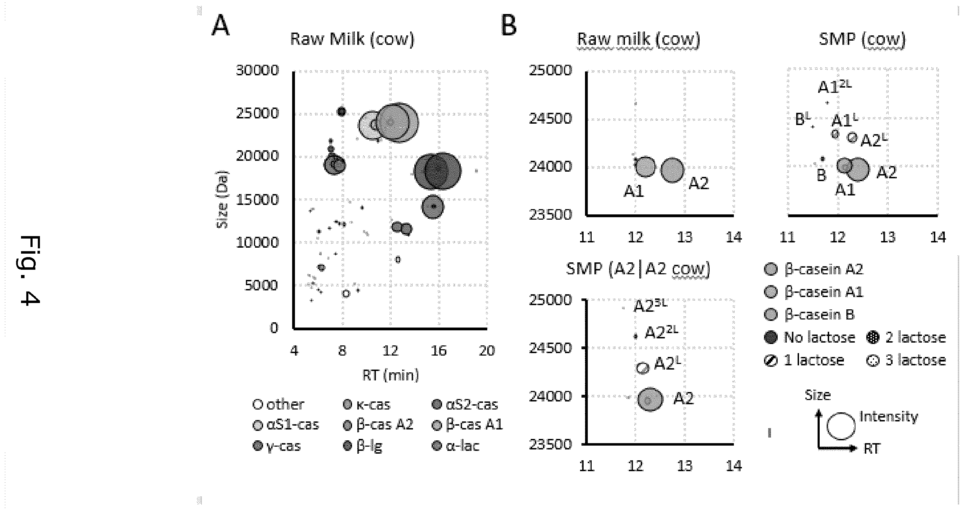

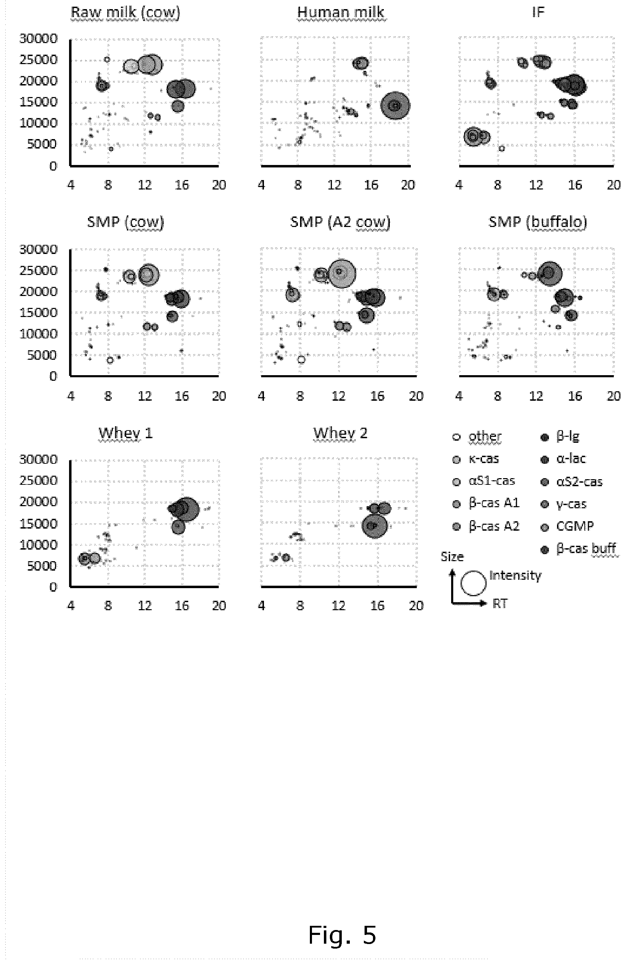

Figure 4A illustrates method output with cow raw milk and highlights where major milk proteins (αS1-, oS2-, β-, κ- and γ-casein, α-lac and β-lg) are detected. Zooming into the β-casein region (Figure 4B) allows detailed analysis of the various β-casein proteoforms including genetic variants and protein modification (process-induced lactosylated adducts are detectable in all skim milk powder samples). Mass accuracy is shown in Table 5 for the raw milk sample. A 1 Da difference may be due to a misattributed monoisotopic signal propagated throughout the deconvolution since a merging tolerance of 1.5 Da is applied between the sliding windows to avoid splitting signals into two masses that are 1 Da apart. The method can be used to profile a wide variety of raw materials and finished products (Figure 5). Interestingly, this method readily detects casein glycomacropeptide (CGMP), the C-terminus part of κ-casein released by rennet enzyme during cheesemaking process, a polypeptide notoriously hard to detect by gel staining or UV profiling.

Table 5: mass accuracy in cow raw milk sample

|  | expected | detected | Δmass | in ppm |
|---|---|---|---|---|
| β-casein A1 (5P) | 24008.16 | 24007.00 | -1.16 | -48.51 |
| β-casein A2 (5P) | 23968.15 | 23967.10 | -1.06 | -44.09 |
| αS1-casein(8P) | 23600.21 | 23599.64 | -0.57 | -24.18 |
| αS2-casein(11P) | 25212.98 | 25212.12 | -0.86 | -34.17 |

(continued)

|  | expected | detected | $\Delta$mass | in ppm |
|---|---|---|---|---|
| $\alpha$-lac | 14176.81 | 14176.87 | 0.06 | 4.44 |
| $\beta$-lg A | 18355.46 | 18355.54 | 0.08 | 4.09 |
| $\beta$-lg B | 18269.42 | 18269.49 | 0.07 | 3.78 |
| $\kappa$-casein A | 19025.53 | 19025.62 | 0.09 | 4.85 |
| $\kappa$-casein B | 18993.58 | 18993.73 | 0.15 | 7.86 |
| $\gamma$2-casein | 11816.25 | 11816.25 | 0.00 | -0.42 |
| $\gamma$3-casein | 11551.10 | 11551.13 | 0.03 | 2.60 |

**Example 3 - Detection of $\beta$-casein A1 in $\beta$-casein A2 Infant formulas**

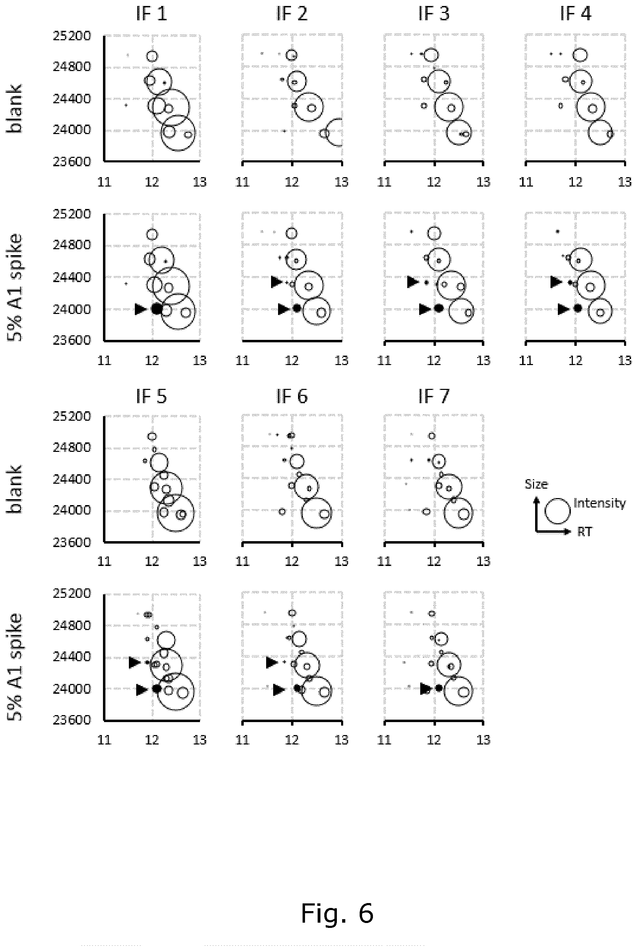

Seven $\beta$-casein A2 infant formulas were spiked with A1|A1 SMP to 5% $\beta$-casein A1 (5 g $\beta$-casein A1 in 100 g of total $\beta$-casein). At this concentration, $\beta$-casein A1 signal was detected in all cases (Figure 6).

**Example 4 - Protein quantification and glycation**

Protein quantification requires not only a suitable standard to establish a calibration curve but also that different proteoforms exhibit similar signal responses. This is necessary to apply the calibration curve to unknown samples given that the standard and the samples are likely to have different proteoform distributions. Alternatively, the method should provide a way to correct for the proteoform distribution.

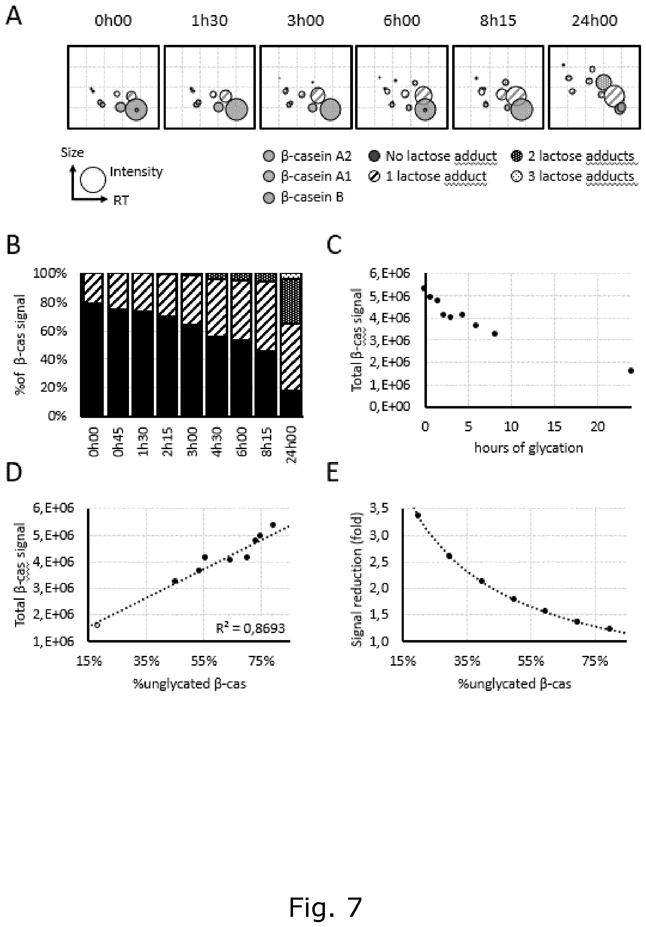

To explore whether protein lactosylation influences MS signal intensity, glycation was induced in a skim milk powder sample by heating as outlined in the materials and methods (Figure 7A). The increase in glycated $\beta$-casein (Figure 7B) was accompanied by a decrease in the total $\beta$-casein signal (Figure 7C). Plotting the total $\beta$-casein signal against the percentage of unglycated $\beta$-casein signal followed a linear relationship (Figure 7D) that was converted into a fold signal reduction (Figure 7E) of the form y = 1 / (ax+b), with a and b corresponding to 0.878 and 0.122, respectively. For $\beta$-casein, a Glycation Correction Factor (GCF) can thus be calculated from the percentage of unglycated $\beta$-casein (%UG$_\beta$) using Equation 1.

$$GCF = \frac{1}{0.878 \times \%UG_\beta + 0.122} \qquad \text{(Equation 1)}$$

Multiplying the measured $\beta$-casein signal by the GCF yields the corrected $\beta$-casein signal ($\beta$cas$_{corr}$), which corresponds to the expected signal if all $\beta$-casein were unglycated (Equation 2).

$$\beta cas_{corr} = \beta cas_{meas} \times GCF \qquad \text{(Equation 2)}$$

**Example 5 - $\beta$-casein quantification**

Quantification was assessed using the sliding windows algorithm. An external calibration curve was established using commercial purified $\beta$-casein. This standard also contained $\alpha$S1-casein, $\kappa$-casein, and various unidentified peptides and protein fragments, most of which were matching $\beta$-casein cleavage products (Figure 8A). The fraction of the total signal attributable to intact $\beta$-casein in the seven most concentrated calibration samples reached 85.6 $\pm$ 0.4 %, and the concentration of standard $\beta$-casein was adjusted with this purity factor. For each concentration, the total $\beta$-casein signal was normalized to that of the average (and corrected for glycation) signal of SMP samples that had been injected in the same series (and in all subsequent quantification experiments to account for variations in MS performance), and normalized values were plotted against the amount of $\beta$-casein loaded on the column. The calibration curve (Figure 8B) showed good linearity ($R^2$ = 0.996) with a random distribution of the residuals. The amount of $\beta$-casein injected on the column (in $\mu$g) can be calculated from this calibration curve using Equation 3:

$$\beta cas_{inj}[\mu g] = \frac{\beta cas_{corr,norm}}{0.0524} \qquad \text{(Equation 3)}$$

where $\beta cas_{corr,norm}$ is the corrected signal for β-casein in the sample (Equation 2) divided by the average of the corrected signals for the skim milk powder samples injected in the same series (Equation 4).

$$\beta cas_{corr,norm} = \frac{\beta cas_{corr}}{\bar{\beta} cas_{SMP,corr}}$$

(Equation 4)

Quantification of β-casein variants in a dairy matrix was further assessed by mixing a skim milk powder containing only the A1 variant with a skim milk powder containing only the A2 type (Figure 8C). The response for β-casein A2 showed good linearity ($R^2$ = 0.996), indicating that the matrix effect was negligible. The measured and expected A1/A2 ratios showed a very strong linear relationship (%A2$_{calc}$ = 1.05 · %A2$_{exp}$, $R^2$ = 0.996). This means that the response factors of both variants are very close and suggests that the calibration curve is also suitable to quantify individual genetic variants. Quantification of β-casein in the two SMP samples yielded an average value of 28.3 g β-casein / 100 g protein, in line with the 27 % value described in the Handbook of milk composition (Swaisgood, 1995) (Table 6).

Table 6: Quantification of β-casein in A1|A1 and A2|A2 SMP

|  | MS signal | | | normalized to SMP | | μg β-casein on column | |
|---|---|---|---|---|---|---|---|
|  | A2\|A2 | A1\|A1 | SMP (n=3) | A2\|A2 | A1\|A1 | A2\|A2 | A1\|A1 |
| Rep1 | 389956856 | 311884671 | 336558582 | 1.16 | 0.93 | 22.1 | 17.7 |
| Rep2 | 374319489 | 319123478 | 336558582 | 1.11 | 0.95 | 21.2 | 18.1 |
| Average | 382138173 | 315504074 | 336558582 | 1.14 | 0.94 | 21.7 | 17.9 |
|  |  |  |  | μg protein on column | | 70.0 | 70.0 |
|  |  |  |  | β-casein content | | 31% | 26% |

**Example 6 - Method performance (β-casein quantification)**

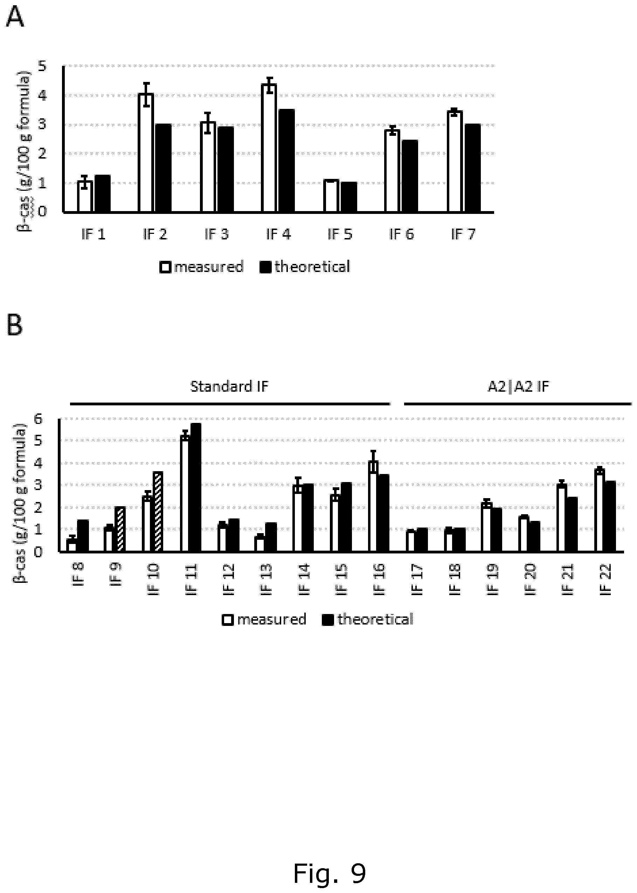

Method performance was assessed in seven infant formulas containing only β-casein A2. Each sample was prepared five different times and analyzed by triplicate injection. The measured amount of β-casein was generally in line (83%-136%) with theoretical values calculated by multiplying the protein content by the casein content and by 33%, which represents the generally accepted proportion of β-casein in the total casein (Swaisgood, 1995) (Figure 9A).

The method was further tested on another series of commercially available infant formulas (either A2-based or manufactured with standard SMP) and shown to be generally in line with the theoretical values (Figure 9B, the %whey is based on assumptions for hatched values). The differences between measured and theoretical β-casein content were the largest for samples with the smallest β-casein content. This might be due to the fact that the less abundant proteoforms become too close to the noise to be measured effectively, in particular in samples with multiple genetic variants such as IF8 and IF13.

**Example 7 - Detection of A1 specific proteose peptones in whey protein concentrate by intact protein analysis**

The six different batches of whey protein concentrates where analysed using the intact protein analysis as described in the methods and materials section using LC-HRMS.

Raw material analysed:

- Three batches of WPC35
- Two batches of WPC28
- One batch of WPC80 (a-lac enriched)

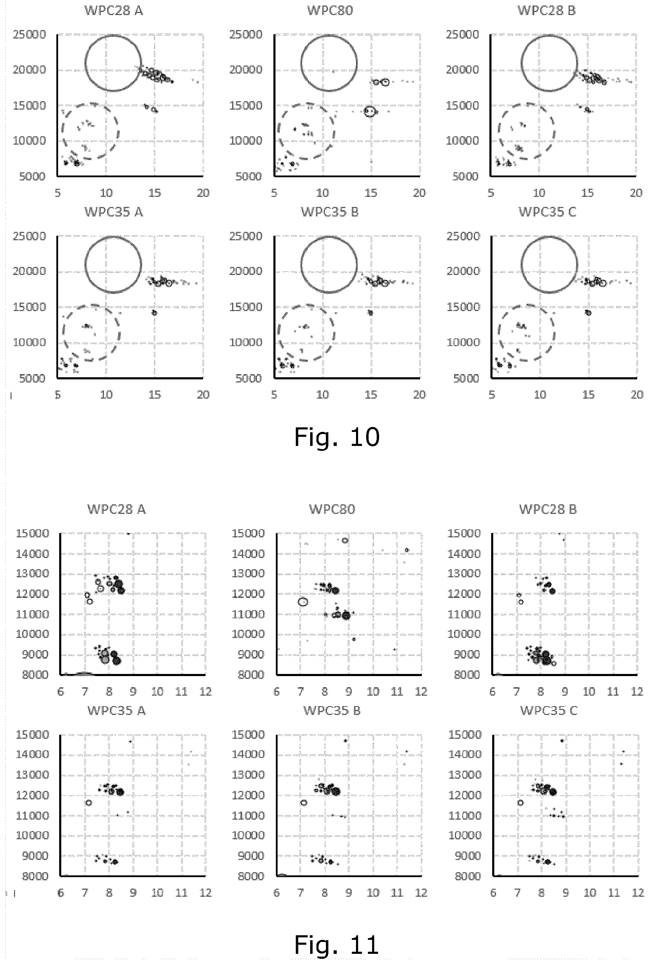

Figure 10 illustrates the separate analyses of the six different whey protein concentrates.

None of the WPC tested showed traces of β-casein as no signals were detected within the circle with the solid perimeter (i.e circle in the upper right corner).

In contrast, all WPCs tested showed signals corresponding to proteose peptones detected as signals within the circle with the dashed perimeter (i.e. circles in the lower left corner).

In addition, the analysis showed that all ingredients contained CGMP (casein glycomacropeptide), which is a casein-derived peptide, identified in the lower left corner of each of the diagrams. Just below the circle with the dashed perimeter

Figure 11 is a close-up of the area within the circle with the dashed perimeter shown in Figure 10. This close-up demonstrates that signals corresponding to the expected masses for a large variety of proteose peptones were found in all ingredients. Black represents β-casein A2 derived proteose peptones, gray represents β-casein A1 derived proteose peptones and white represent unassigned compounds;
Some of these peptones are : PP5 A1 1-105, PP5 A1 1-107, PP5 A2 1-105, PP5 A2 1-107, bcas1P A1 29-105 (i.e. PP8s A1 29-105), bcas1P A1 29-107 (i.e. PP8s A1 29-107), bcas1p A2 29-105 (i.e. PP8s A2 29-105) and bcas1P A2 29-107 (PP8s A2 29-107).

No gamma-casein was detected in the WPC tested.

WPC80 did not contain PP8 slow (29-105/7). Probably due to the ultrafiltration step in the process of preparing WPC80, which would likely remove PP8 slow.

Both in Figure 10 and Figure 11 several signals (shown in white) both within and outside of the circle with the dashed perimeter in Figure 10 relates to peptides remaining identification.

Accordingly, proteose peptones from β-casein A1 (and A2) were found in all whey raw material tested. Hence, they will be present in finished products and are extremely likely to be responsible for the high A1 signal picked up in all finished products tested.

**Example 8** - **Detection of A1 specific proteose peptones in finished products by intact protein analysis**

Different finished products being different infant formulas of different brands were analysed for the detection of specific proteose peptones. All of the infant formulas are considered A2 finished products.

The finished products were dissolved and analysed by LC-HRMS as described in the materials and methods section above.

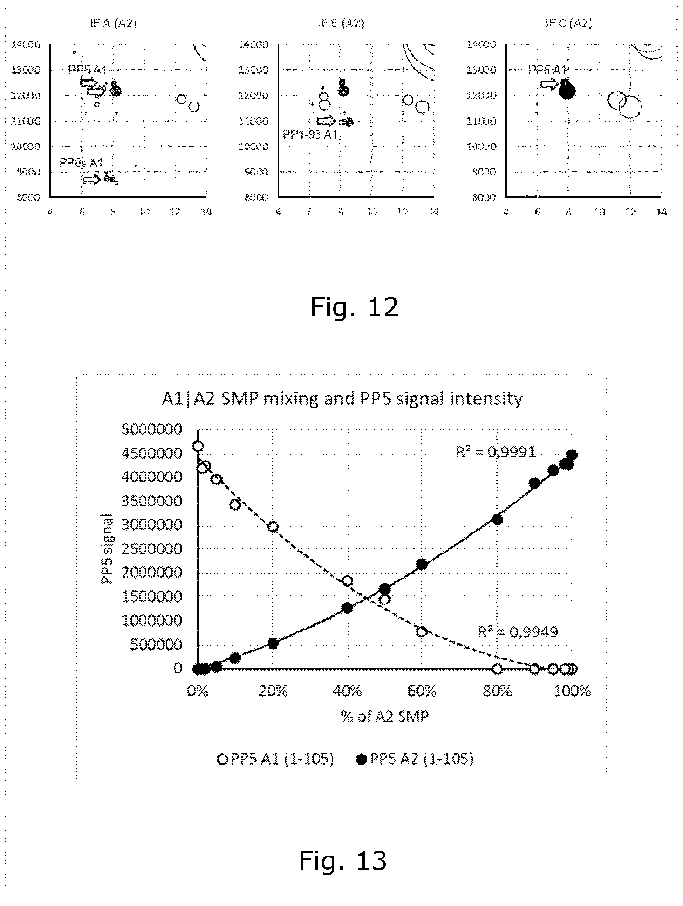

Figure 12 shows that proteose peptones were found in all of the tested batches even though the proteose peptone profile differed among the finished products.

A1 β-casein derived proteose peptones are discovered in all of the infant formulas tested even though they are based on milk with A2 β-casein.

**Example 9** - **Quantification of A1 and A2 specific proteose peptones by intact protein analysis**

Relative quantification of PP5 A1 and PP5 A2 in a dairy matrix was assessed by mixing a skim milk powder containing only the A1 variant with a skim milk powder containing only the A2 type (Figure 13). The responses for PP5 A2 and PP5 A1 showed good dose response having quadratic regression ($R^2$ = 0.999 and $R^2$ = 0.995, respectively), indicating that the matrix effect was negligible. This means that the response factors of both variants are very close and suggests that the calibration curve is also suitable to quantify individual genetic variants.

Conclusively, it is shown that specific proteose peptones can be quantified using the intact protein analysis even in a matrix.

**Example 10** - **Detection of A1 specific proteose peptones in finished products by tryptic digest**

Different finished products being either standard skimmed milk powder (i.e. standard SMP), skimmed milk powder containing only the A2 version of β-casein or different infant formulas of different brands and for different age-groups. All of the infant formulas are considered A2 finished products.

The finished products were dispersed to 3.5 % (w/v) protein in Tris 50 mM + 6M urea and mixed on an orbital shaker for one hour. 200 μL sample were diluted with 1000 μL ammonium bicarbonate 100 mM and vortexed. To 100 μL of the diluted solution, 10 μL of DTT 45 mM were added. Samples were incubated at 60 °C for 30 minutes, cooled to RT and quickly spun to collect evaporation droplets. 10 μL iodoacetamide 100 mM (in ammonium bicarbonate 100 mM) were added; samples were incubated 30 minutes at room temperature in the dark. 6 μL trypsin 0.2 μg/μL were added and samples were incubated overnight at 37 °C. 6 μL formic acid 10% were added to stop digestion, solutions were centrifuged at 14'000g for 10 minutes; the liquid phase was transferred into a vial for injection and analysed by LC-MS as described in the materials and methods section above.

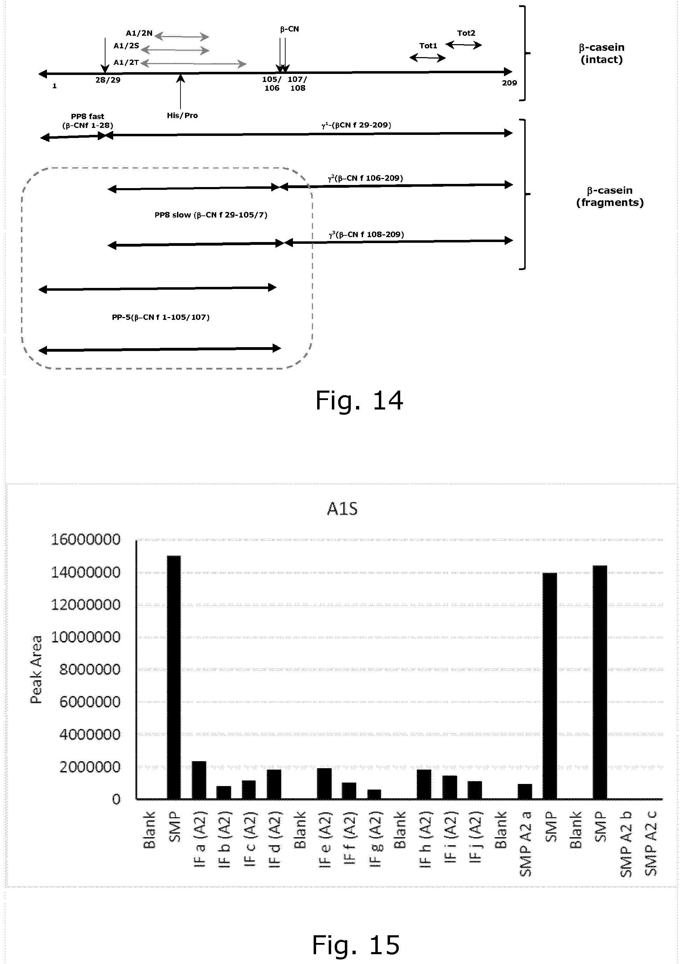

The analysis focused on the detection of A1S peptide i.e. an A1-specific peptide having a position as illustrated in Figure 14. As evident from the position of the A1S peptide it can be distinguished from the A2S peptide as it covers the area of β-casein including the amino acid 67.

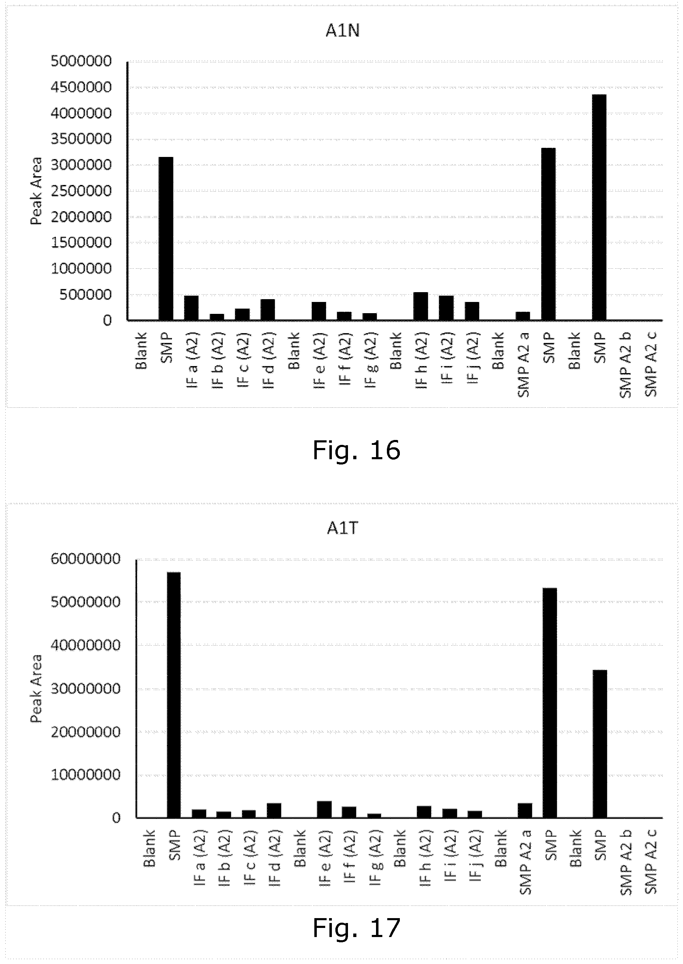

Figure 15 illustrates that no A1S is detected in A2 SMP samples. A1S was detected in one A2 SMP sample, which proved subsequently to mistakenly contain β-casein A1. However, the peptide is significantly expressed in standard SMP. Surprisingly, a strong signal was detected for the peptide A1S in all the infant formulas tested. Similar results were obtained for the peptides A1N (Figure 16) and A1T (Figure 17).

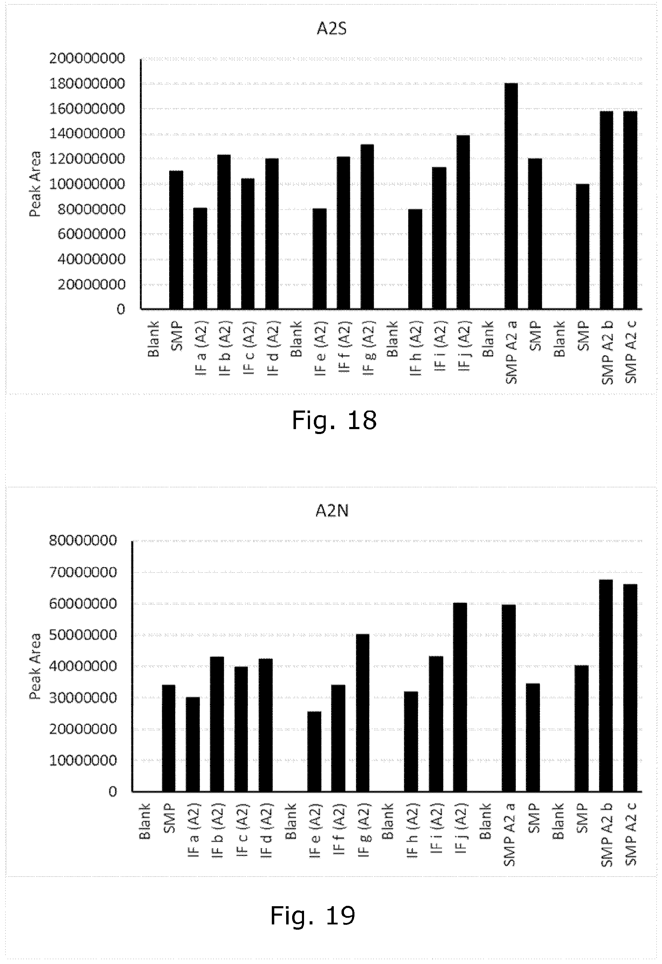

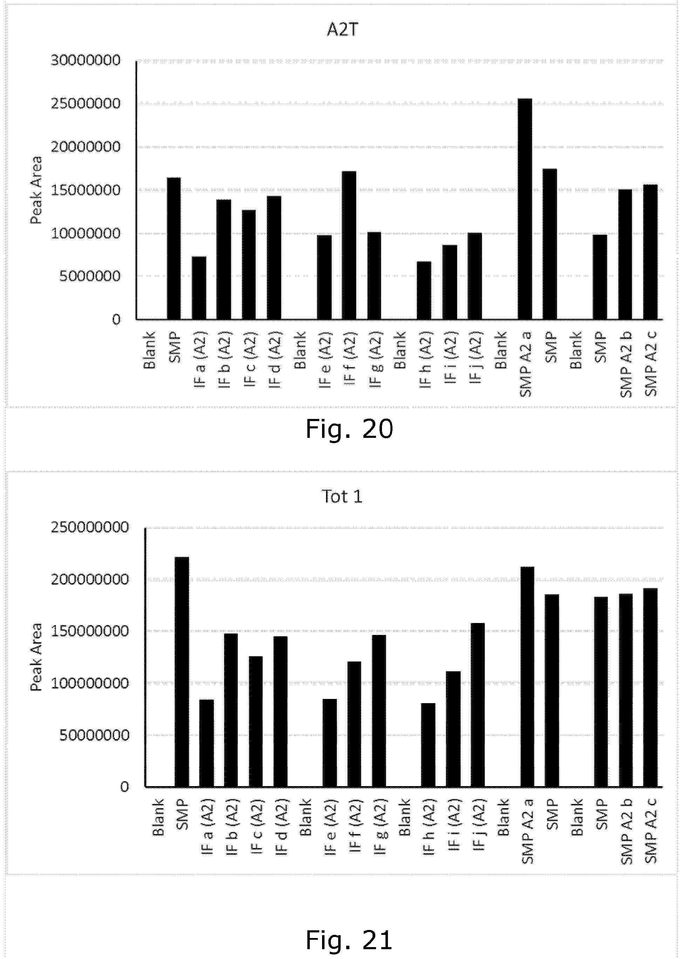

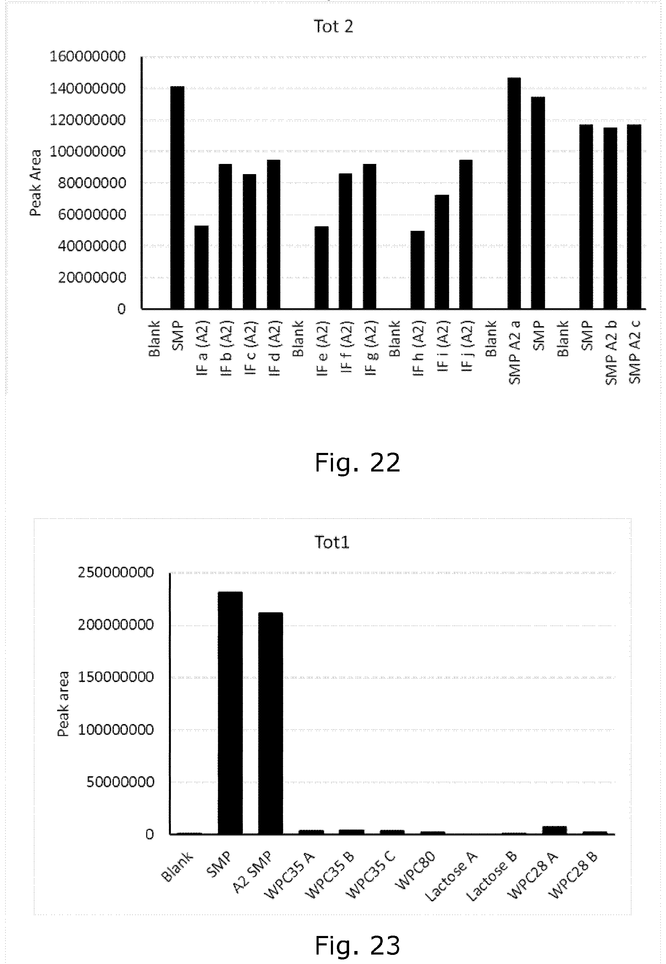

As expected the peptides A2S (Figure 18), A2N (Figure 19), A2T (Figure 20), Tot 1 (Figure 21) and Tot 2 (Figure 22) showed strong signals in all of the areas tested.

**Example 11 - Detection of A1 specific proteose peptones in whey protein concentrate by tryptic digest**

Different batches of whey protein concentrates where analysed together with skimmed milk powder both standard and skimmed milk powder containing only the A2 version of β-casein.

Raw materials (RM) analysed:

- Three batches of WPC35
- Two batches of WPC28
- One batch of WPC80 (a-lac enriched)
- Two different commercially available lactose ingredients

The products were dissolved and exposed to tryptic digestion before being analysed by LC-MS as described in the materials and methods section above.

The analyses focused on the detection of Tot1 and Tot2 peptides i.e. common to β-casein to A1 and A2, A1S peptide i.e. an A1-specific peptide, A1N peptide i.e. an A1-specific peptide, A1T peptide i.e. an A1-specific peptide, A2N peptide i.e. an A2-specific peptide, A2T peptide i.e. an A2-specific peptide and A2S peptide i.e. an A2-specific peptide. The position of the peptides is illustrated in Figure 14.

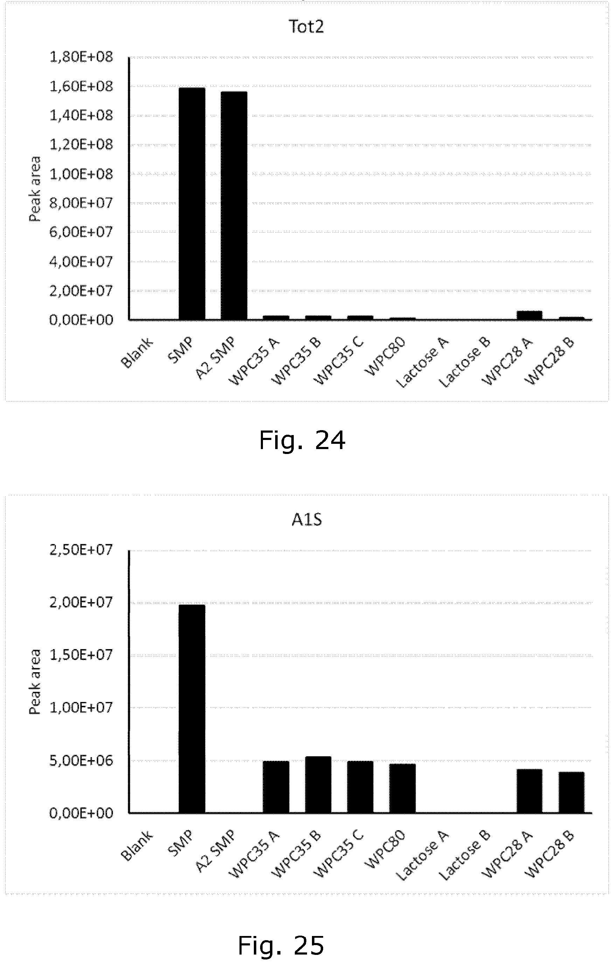

Figures 23 and 24 illustrate the results obtained when determining the amount of Tot1 and Tot2 peptide, respectively. It is a common (to A1 and A2) peptide but also a peptide present only in intact β-casein and γ-casein. It is shown that the signal for the common β-casein peptide is severely reduced in all whey ingredients, and absent in lactose ingredients but present in the SMP and A2 SMP as would be expected.

This indicates that very little intact β-casein or γ-casein is present in the RM (~1%) compared to SMP even when an equivalent protein content was used, as expected.

Figure 25 illustrates the results obtained when determining the amount of A1S peptide. It is shown that the signal for the A1S peptide is strongest for the SMP but lacking for A2 SMP as expected. Also the lactose samples does not show any signal. For the whey protein samples, a surprising amount of about a fourth of the amount observed in SMP was determined.

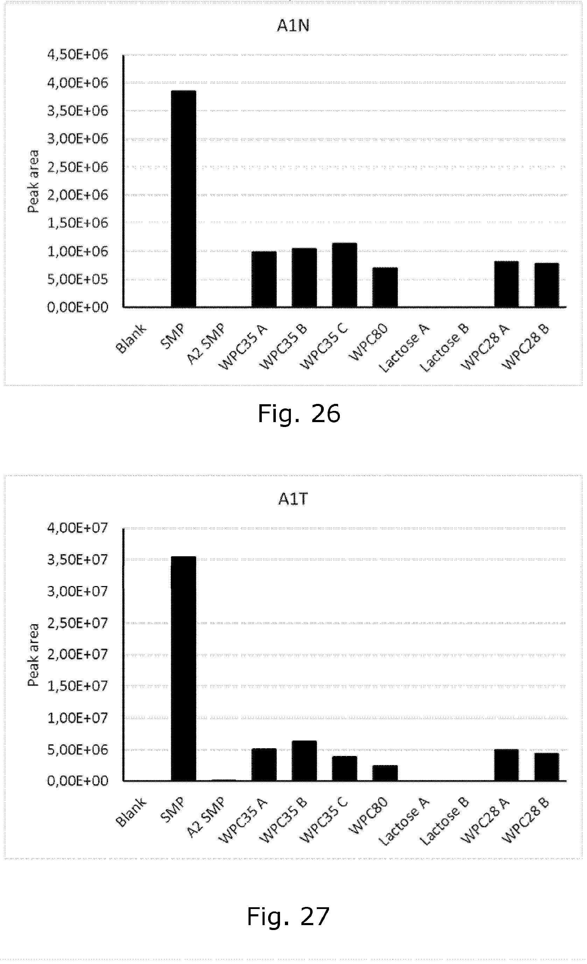

Similar results were obtained when the samples were tested for the presence of two other A1-specific peptides A1N and A1T (Figure 26 & Figure 27).

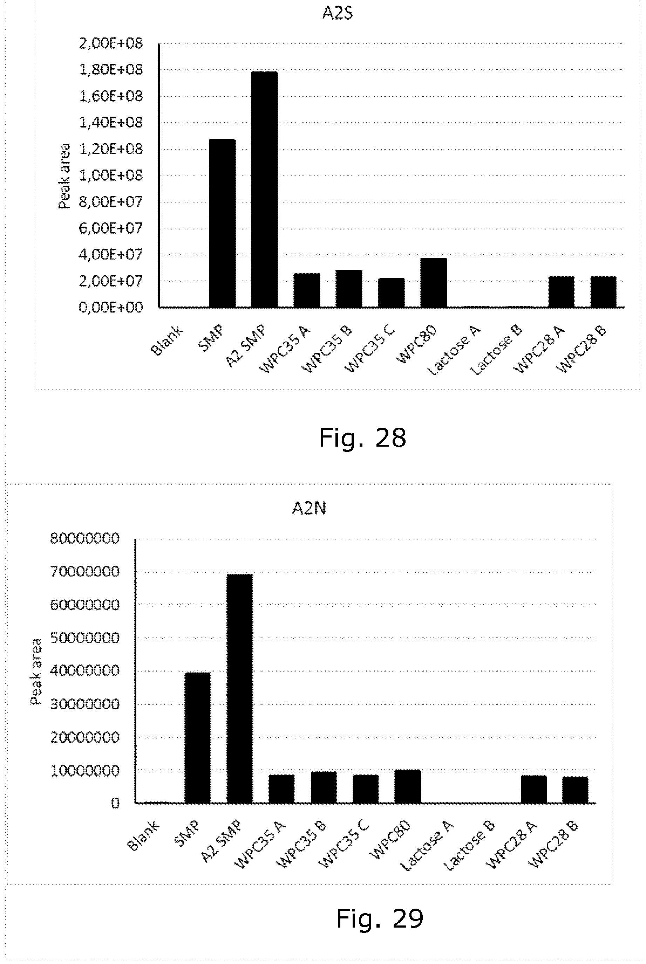

Figure 28 illustrates the results obtained when determining the amount of A2S. It is shown that the signal for the A2S (A2-specific) peptide is strongest for the A2 SMP but weaker for the SMP as expected. The lactose samples do not show any signal as expected. For the whey protein samples, a surprising amount of about a fourth of the amount observed in SMP was determined.

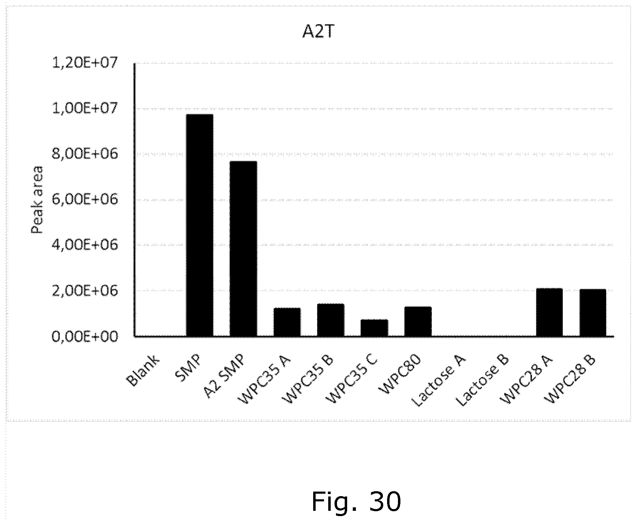

Similar results were obtained when the samples were tested for the presence of two other A2-specific peptides A2N and A2T (Figures 29 and 30).

Accordingly, it can be concluded from the above results that in all whey RM analyzed, the signal intensity of the β-casein common peptide is severely reduced (~100x) as compared to SMP. Also, the A1- and A2-specific peptides are only ~4-5x reduced compared to SMP. Hence, there is a selective enrichment of the A1- and A2-specific peptides.

Thus, using the tryptic digest method and determining the different peptide, it would seem that the whey ingredients still contain ~20% of β-casein fragments (compared to SMP), which may at least partly be known proteose peptones such as PP5 and PP8s.

**Example 12 - Reduction of proteose peptones in whey protein fractions**

In order to obtain a reduced whey protein fraction, three batches of WPC35 where the presence of proteose peptones were determined in Example 9, are subjected to a gel filtration.

The three batches of WPC35 are further purified by gel filtration on a column (550 x 22 mm) of Sephadex G-75 made up in and equilibrated with volatile 0.1 M $NH_4HCO_3$ (pH 8.0-8.5) buffer.

The 0.5 g-0.7 g of each of the batches are dissolved in 5-7 ml buffer (with addition of a few ml of 1 M NaOH to neutralize residual traces of TCA and aid dissolution) and applied to the column. Flow rate is adjusted to 0.5 ml/min and 5 ml fractions are collected.

The fractions are analysed by the detection and quantification method as described herein i.e. LC-HRMS according to the materials and methods section. Fractions without proteose peptones PP5 and PP8 slow are pooled to form a reduced whey protein fraction.

**References**

Bonfatti, V. et al. J Chromatogr A, 2008; 1195(1-2):101-106.
de Jong et al. J Chromatogr A, 1993; 652(1):207-213.
EFSA Scientific Report, Scientific Report of EFSA prepared by a DATEX Working Group on the potential health impact of b-casomorphins and related peptides. 2009;231;1-107.
Fenaille, F. et al. Rapid Commun Mass Spectrom, 2003; 17(13):1483-1492.
Fenaille, F. et al. International Dairy Journal, 2006; 16(7):728-739.
Feng, P. et al. J AOAC Int, 2017; 100(2):510-521.
Frederiksen, P. D. et al. J Dairy Sci, 2011; 94(10):4787-4799.
Ho, S. et al. Eur. J. Clin. Nutr. 2014, 68, 994-1000.
Karamoko, G. et al. Biotechnologie, Agronomie, Société et Environnement, 2013 ; 17(2):373-382.
Poulsen, N. A et al. Acta Agriculturae Scandinavica, Section A - Animal Science, 2016; 66(4): 190-198.
Roberfroid MB. J Nutr. 2007; 137: 830S.
Salminen S et al. Trend Food Sci. Technol., 1999;10 107-110.
Swaisgood, H. E. Handbook of Milk Composition. R. G. Jensen, ed. Academic Press, San Diego 1995; 464-468.
Swaisgood. Developments in Dairy Chemistry. Fox (Ed), Proteins, vol. 1, 1982; 63-110.
Vallejo-Cordoba, B. J Capillary Electrophor, 1997; 4(5):219-224.
Visser, S. et al. J Chromatogr A, 1995; 711(1):141-150.

**Sequence listing**

SEQ ID NO. 1 (amino acid sequence of A2 β-casein):

RELEELNVPG EIVESLSSSE ESITRINKKI EKFQSEEQQQ TEDELQDKIH PFAQTQSLVY

PFPGPIPNSL PQNIPPLTQT PVVVPPFLQP EVMGVSKVKE AMAPKHKEMP FPKYPVEPFT

ESQSLTLTDV ENLHLPLPLL QSWMHQPHQP LPPTVMFPPQ SVLSLSQSKV LPVPQKAVPY

PQRDMPIQAF LLYQEPVLGP VRGPFPIIV

SEQ ID NO.2 (amino acid sequence of A1 β-casein):

RELEELNVPG EIVESLSSSE ESITRINKKI EKFQSEEQQQ TEDELQDKIH PFAQTQSLVY

PFPGPIHNSL PQNIPPLTQT PVVVPPFLQP EVMGVSKVKE AMAPKHKEMP FPKYPVEPFT

ESQSLTLTDV ENLHLPLPLL QSWMHQPHQP LPPTVMFPPQ SVLSLSQSKV LPVPQKAVPY

PQRDMPIQAF LLYQEPVLGP VRGPFPIIV

SEQ ID NO.3 (amino acid sequence of A1N): AQTQSLVYPF PGPIHN

SEQ ID NO.4 (amino acid sequence of A2N): AQTQSLVYPF PGPIPN

SEQ ID NO.5 (amino acid sequence of Tot2): AVPYPQR

SEQ ID NO.6 (amino acid sequence of A1S): IHPFAQTQSL VYPFPGPIHN

SEQ ID NO.7 (amino acid sequence of A1T):

IHPFAQTQSL VYPFPGPIHN SLPQNIPPLT QTPVVVPPFL QPEVMGVSK

SEQ ID NO.8 (amino acid sequence of A2S): IHPFAQTQSL VYPFPGPIPN

SEQ ID NO.9 (amino acid sequence of A2T):
IHPFAQTQSL VYPFPGPIPN SLPQNIPPLT QTPVVVPPFL QPEVMGVSK

SEQ ID NO.10 (amino acid sequence of Tot1): VLPVPQK

## Claims

1. A method for producing a whey protein fraction having a reduced β-casein derived proteose peptone content, said method comprising the steps of

    (i) providing a whey protein fraction;
    (ii) determining and quantifying said β-casein derived proteose peptones in said whey protein fraction; and
    (iii) selecting said whey protein fraction having at the most 10% by weight of β-casein derived proteose peptones based on the total protein in said whey protein fraction forming a selected whey protein fraction, or reducing the β-casein derived proteose peptone content in said whey protein fraction to a concentration of at the most 10% by weight based on total protein in the whey protein fraction forming a reduced whey protein fraction by gel filtration,

    Wherein determining and quantifying said β-casein derived proteose peptones in said whey protein fraction comprising the steps of:

    (i) providing a dairy-based product to be analysed;
    (ii) subjecting said product using liquid chromatography - mass spectrometry analysis;
    (iii) determining and/or quantifying said β-casein derived proteose peptones and/or β-casein in said product by detecting compounds of defined m/z values or deconvoluting one or more mass spectrometry spectra to calculate monoisotopic masses.

2. A method for producing a nutritional composition having a reduced β-casein derived proteose peptone content, said method comprising the steps of

    (i) providing a selected whey protein fraction or a reduced whey protein fraction as described in claim 1;
    (ii) preparing said nutritional composition with a reduced proteose peptone content from said selected whey protein fractions, said reduced whey protein fractions or a mixture hereof.

## Patentansprüche

1. Verfahren zum Produzieren einer Molkeproteinfraktion, die einen reduzierten Gehalt an β-Casein-stammendem Proteosepepton aufweist, das Verfahren umfassend die Schritte

    (i) Bereitstellen einer Molkeproteinfraktion;
    (ii) Bestimmen und Quantifizieren der β-Casein-stammenden Proteosepeptone in der Molkeproteinfraktion; und
    (iii) Auswählen der Molkeproteinfraktion, die zu höchstens 10 Gew.-% β-Casein-stammende Proteosepeptonen, bezogen auf das Gesamtprotein in der Molkeproteinfraktion aufweist, wobei eine ausgewählte Molkeproteinfraktion ausgebildet wird, oder Reduzieren des Gehalts an β-Casein-stammendem Proteosepepton in der Molkeproteinfraktion auf eine Konzentration von höchstens 10 Gew.-%, bezogen auf das Gesamtprotein in der Molkeproteinfraktion, wobei durch Gelfiltration eine reduzierte Molkeproteinfraktion ausgebildet wird,

    wobei Bestimmen und Quantifizieren der β-Casein-stammenden Proteosepeptone in der Molkeproteinfraktion die Schritte umfasst:

    (i) Bereitstellen eines zu analysierenden milchbasierten Erzeugnisses;
    (ii) Unterziehen des Erzeugnisses einer Analyse mittels Flüssigkeitschromatographie und Massenspektrometrie ;

(iii) Bestimmen und/oder Quantifizieren der β-Casein-stammenden Proteosepeptonen und/oder des β-Caseins in dem Erzeugnis durch Detektieren von Verbindungen mit definierten m/z-Werten oder durch Dekonvolution eines oder mehrerer Massenspektrometriespektren, um monoisotopische Massen zu berechnen.

**2.** Verfahren zum Produzieren einer Nährstoffzusammensetzung, die einen reduzierten Gehalt an β-Casein-stammendem Proteosepepton aufweist, das Verfahren umfassend die Schritte

(i) Bereitstellen einer ausgewählten Molkeproteinfraktion oder einer reduzierten Molkenproteinfraktion wie in Anspruch 1 beschrieben;

(ii) Herstellen der Nährstoffzusammensetzung mit einem reduzierten Gehalt an Proteosepepton aus den ausgewählten Molkeproteinfraktionen, den reduzierten Molkeproteinfraktionen oder einer Mischung davon.

**Revendications**

**1.** Procédé de production d'une fraction de protéine de lactosérum ayant une teneur réduite en protéose-peptone dérivée de la β-caséine, ledit procédé comprenant les étapes consistant à

(i) fournir une fraction de protéine de lactosérum ;

(ii) déterminer et quantifier lesdites protéose-peptones dérivées de la β-caséine dans ladite fraction de protéines de lactosérum ; et

(iii) sélectionnerladite fraction de protéines de lactosérum ayant au plus 10 % en poids de protéose-peptones dérivées de la β-caséine sur la base de la protéine totale dans ladite fraction de protéines de lactosérum formant une fraction de protéine de lactosérum sélectionnée, ou réduire la teneur en protéose-peptones dérivées de la β-caséine dans ladite fraction de protéines de lactosérum à une concentration d'au plus 10 % en poids sur la base de la protéine totale dans la fraction de protéines de lactosérum formant une fraction de protéine de lactosérum réduite par filtration sur gel,

dans lequel la détermination et la quantification desdites protéose-peptones dérivées de la β-caséine dans ladite fraction de protéine de lactosérum comprennent les étapes consistant à :

(i) fournir un produit laitier à analyser ;

(ii) soumettre ledit produit à une analyse par chromatographie en phase liquide - spectrométrie de masse ;

(iii) déterminer et/ou quantifier lesdites protéose-peptones dérivées de la β-caséine et/ou ladite β-caséine dans ledit produit en détectant des composés de valeurs m/z définies ou en déconvoluant un ou plusieurs spectres de spectrométrie de masse pour calculer les masses mono-isotopiques.

**2.** Procédé de production d'une composition nutritionnelle ayant une teneur réduite en protéose-peptone dérivée de la β-caséine, ledit procédé comprenant les étapes consistant à

(i) fournir une fraction sélectionnée de protéines de lactosérum ou une fraction réduite de protéines de lactosérum tel que décrit dans la revendication 1 ;

(ii) préparer ladite composition nutritionnelle ayant une teneur réduite en protéose-peptone à partir desdites fractions sélectionnées de protéine de lactosérum, desdites fractions réduites de protéine de lactosérum ou d'un mélange de celles-ci.

| | 59 | 60 | 61 | 62 | 63 | 64 | 65 | 66 | 67 | 68 | 69 | 70 | 71 |
|---|---|---|---|---|---|---|---|---|---|---|---|---|---|
| A2 beta-casein | Val | Tyr | Pro | Phe | Pro | Gly | Pro | Ile | **Pro** | Asn | Ser | Leu | Pro |
| A1 beta-casein | Val | Tyr | Pro | Phe | Pro | Gly | Pro | Ile | **His** | Asn | Ser | Leu | Pro |

# Fig. 1

| | 59 | 60 | 61 | 62 | 63 | 64 | 65 | 66 | 67 | 68 | 69 | 70 | 71 |
|---|---|---|---|---|---|---|---|---|---|---|---|---|---|
| A2 beta-casein | Val | Tyr | Pro | Phe | Pro | Gly | Pro | Ile | **Pro** | Asn | Ser | Leu | Pro |
| A1 beta-casein | Val | | | | | | | | **His** | Asn | Ser | Leu | Pro |

Tyr Pro Phe Pro Gly Pro Ile
Beta-casomorphin-7 (BCM-7)

# Fig. 2

**A**

**B**

**C**

Fig. 3

Fig. 4

Fig. 5

Fig. 6

Fig. 7

Fig. 8

A

B

Fig. 9

Fig. 10

Fig. 11

## Fig. 12

## Fig. 13

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

Fig. 28

Fig. 29

Fig. 30

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2098122 A
- EP 1234507 A1

**Non-patent literature cited in the description**

- *EFSA Scientific Report,* 2009
- **BONFATTI, V et al.** *J Chromatogr A,* 2008, vol. 1195 (1-2), 101-106
- **JONG et al.** *J Chromatogr A,* 1993, vol. 652 (1), 207-213
- Scientific Report of EFSA prepared by a DATEX Working Group on the potential health impact of b-casomorphins and related peptides. *EFSA Scientific Report,* 2009, vol. 231, 1-107
- **FENAILLE, F et al.** *Rapid Commun Mass Spectrom,* 2003, vol. 17 (13), 1483-1492
- **FENAILLE, F. et al.** *International Dairy Journal,* 2006, vol. 16 (7), 728-739
- **FENG, P. et al.** *J AOAC Int,* 2017, vol. 100 (2), 510-521
- **FREDERIKSEN, P. D. et al.** *J Dairy Sci,* 2011, vol. 94 (10), 4787-4799
- **HO, S. et al.** *Eur. J. Clin. Nutr.,* 2014, vol. 68, 994-1000
- **KARAMOKO, G. et al.** *Biotechnologie, Agronomie, Société et Environnement,* 2013, vol. 17 (2), 373-382
- **POULSEN, N. A et al.** *Acta Agriculturae Scandinavica, Section A - Animal Science,* 2016, vol. 66 (4), 190-198
- **ROBERFROID MB.** *J Nutr.,* 2007, vol. 137, 830S
- **SALMINEN S et al.** *Trend Food Sci. Technol.,* 1999, vol. 10, 107-110
- **SWAISGOOD, H. E.** Handbook of Milk Composition. Academic Press, 1995, 464-468
- Developments in Dairy Chemistry. **SWAISGOOD.** Proteins. 1982, vol. 1, 63-110
- **VALLEJO-CORDOBA, B.** *J Capillary Electrophor,* 1997, vol. 4 (5), 219-224
- **VISSER, S. et al.** *J Chromatogr A,* 1995, vol. 711 (1), 141-150